(19) Europäisches Patentamt
European Patent Office
Office européen des brevets

(11) EP 1 431 387 B1

(12) # EUROPEAN PATENT SPECIFICATION

(45) Date of publication and mention of the grant of the patent:
**27.02.2008 Bulletin 2008/09**

(51) Int Cl.:
*C12N 9/22* (2006.01) *C12N 15/55* (2006.01)
*C12N 1/16* (2006.01)

(21) Application number: **03028861.7**

(22) Date of filing: **16.12.2003**

(54) **Heat-labile desoxyribonculease I variants**

Hitzelabile Desoxyribonuklease I-Varianten

Variants desoxyribonuclease I instable à la chaleur

(84) Designated Contracting States:
**AT BE BG CH CY CZ DE DK EE ES FI FR GB GR HU IE IT LI LU MC NL PT RO SE SI SK TR**

(30) Priority: **20.12.2002 EP 02028558**
**20.01.2003 EP 03001214**
**21.01.2003 US 441550 P**

(43) Date of publication of application:
**23.06.2004 Bulletin 2004/26**

(73) Proprietors:
- **Roche Diagnostics GmbH**
  **68305 Mannheim (DE)**
  Designated Contracting States:
  **DE**
- **F. HOFFMANN-LA ROCHE AG**
  **4070 Basel (CH)**
  Designated Contracting States:
  **AT BE BG CH CY CZ DK EE ES FI FR GB GR HU IE IT LI LU MC NL PT RO SE SI SK TR**

(72) Inventors:
- **Mueller, Rainer, Dr.**
  **82377 Penzberg (DE)**
- **Kirschbaum, Thomas, Dr.**
  **82377 Penzberg (DE)**
- **Suppmann, Bernhard, Dr.**
  **82362 Weilheim (DE)**
- **Schoen, Helmut, Dr.**
  **82377 Penzberg (DE)**
- **Engh, Richard, Dr.**
  **82234 Wessling (DE)**
- **Hoffmann, Artur, Dr.**
  **82515 Wolfratshausen (DE)**
- **Thalhofer, Johan-Peter, Dr.**
  **82362 Weilheim (DE)**
- **Siedel, Joachim, Dr.**
  **82347 Bernried (DE)**
- **Engel, Wolf-Dieter, Dr.**
  **82340 feldafing (DE)**

(56) References cited:
**US-A1- 2002 042 052**

- **HOSOMI O ET AL: "Molecular cloning of cDNA encoding Xenopus laevis deoxyribonuclease I." DNA SEQUENCE: THE JOURNAL OF DNA SEQUENCING AND MAPPING. SWITZERLAND 2000, vol. 11, no. 3-4, 2000, pages 247-255, XP009008235 ISSN: 1042-5179**
- **CHEN C-Y ET AL: "Cloning, sequencing and expression of a cDNA encoding bovine pancreatic deoxyribonuclease I in Escherichia coli: purification and characterization of the recombinant enzyme" GENE: AN INTERNATIONAL JOURNAL ON GENES AND GENOMES, ELSEVIER SCIENCE PUBLISHERS, BARKING, GB, vol. 206, no. 2, 12 January 1998 (1998-01-12), pages 181-184, XP004108894 ISSN: 0378-1119**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

SEQUENCE LISTING

<110> Roche Diagnostics GmbH
F. Hoffmann-La Roche AG

<120> Heat-labile desoxyribonuclease I variants

<130> 21551 EP

<140>
<141>

<160> 40

<170> PatentIn Ver. 2.1

<210> 1
<211> 783
<212> DNA
<213> Artificial Sequence

<220>
<223> Description of Artificial Sequence:sequence encoding the wild-type bovine pancreatic DNase I without signal peptide

<220>
<221> CDS
<222> (1)..(780)

<400> 1

```
ttg aag att gct gct ttc aac att aga act ttc ggt gaa act aaa atg     48
Leu Lys Ile Ala Ala Phe Asn Ile Arg Thr Phe Gly Glu Thr Lys Met
  1               5                  10                  15

tct aac gct act ttg gca tct tac atc gtt aga att gtc aga aga tat     96
Ser Asn Ala Thr Leu Ala Ser Tyr Ile Val Arg Ile Val Arg Arg Tyr
             20                  25                  30

gat atc gtt tta att caa gaa gtt aga gac tct cac ttg gtt gca gtt    144
Asp Ile Val Leu Ile Gln Glu Val Arg Asp Ser His Leu Val Ala Val
         35                  40                  45

ggt aaa ttg tta gac tac ttg aac caa gat gac cca aac act tac cac    192
Gly Lys Leu Leu Asp Tyr Leu Asn Gln Asp Asp Pro Asn Thr Tyr His
     50                  55                  60

tac gtt gtt tct gaa cca ttg ggt aga aac tct tac aaa gaa aga tac    240
Tyr Val Val Ser Glu Pro Leu Gly Arg Asn Ser Tyr Lys Glu Arg Tyr
 65                  70                  75                  80

tta ttc ttg ttc aga cca aac aaa gtt tca gtt ttg gat act tac caa    288
Leu Phe Leu Phe Arg Pro Asn Lys Val Ser Val Leu Asp Thr Tyr Gln
                 85                  90                  95

tac gac gac ggt tgc gaa tct tgt ggt aac gat tct ttc tcc aga gaa    336
Tyr Asp Asp Gly Cys Glu Ser Cys Gly Asn Asp Ser Phe Ser Arg Glu
            100                 105                 110
```

```
cct gct gtt gtt aaa ttc tca tca cac tct acc aag gtt aaa gag ttc    384
Pro Ala Val Val Lys Phe Ser Ser His Ser Thr Lys Val Lys Glu Phe
        115                 120                 125

gct atc gtt gct ttg cat tct gct cct tct gac gct gtt gct gaa att    432
Ala Ile Val Ala Leu His Ser Ala Pro Ser Asp Ala Val Ala Glu Ile
    130                 135                 140

aac tct ttg tac gac gtt tac tta gat gtt caa cag aaa tgg cac ttg    480
Asn Ser Leu Tyr Asp Val Tyr Leu Asp Val Gln Gln Lys Trp His Leu
145                 150                 155                 160

aac gac gtc atg ttg atg ggt gac ttt aac gct gat tgc tct tat gtt    528
Asn Asp Val Met Leu Met Gly Asp Phe Asn Ala Asp Cys Ser Tyr Val
                165                 170                 175

act tct tct caa tgg tct tca att aga ttg aga aca tct tca act ttc    576
Thr Ser Ser Gln Trp Ser Ser Ile Arg Leu Arg Thr Ser Ser Thr Phe
            180                 185                 190

caa tgg tta att cct gat tcc gct gat acc act gct act agt acc aac    624
Gln Trp Leu Ile Pro Asp Ser Ala Asp Thr Thr Ala Thr Ser Thr Asn
        195                 200                 205

tgt gct tac gat aga atc gtt gtt gct gga tca tta ttg caa tct tct    672
Cys Ala Tyr Asp Arg Ile Val Val Ala Gly Ser Leu Leu Gln Ser Ser
    210                 215                 220

gtt gtc cca ggt tca gcg gcc cct ttc gat ttc caa gct gca tat ggt    720
Val Val Pro Gly Ser Ala Ala Pro Phe Asp Phe Gln Ala Ala Tyr Gly
225                 230                 235                 240

ttg tct aat gaa atg gct tta gcc att tct gat cac tac cca gtt gaa    768
Leu Ser Asn Glu Met Ala Leu Ala Ile Ser Asp His Tyr Pro Val Glu
                245                 250                 255

gtc aca ttg aca taa                                                783
Val Thr Leu Thr
            260
```

<210> 2
<211> 260
<212> PRT
<213> Artificial Sequence
<223> Description of Artificial Sequence:sequence encoding the wild-type bovine pancreatic DNase I without signal peptide

<400> 2

```
Leu Lys Ile Ala Ala Phe Asn Ile Arg Thr Phe Gly Glu Thr Lys Met
1               5                   10                  15

Ser Asn Ala Thr Leu Ala Ser Tyr Ile Val Arg Ile Val Arg Arg Tyr
            20                  25                  30

Asp Ile Val Leu Ile Gln Glu Val Arg Asp Ser His Leu Val Ala Val
        35                  40                  45

Gly Lys Leu Leu Asp Tyr Leu Asn Gln Asp Asp Pro Asn Thr Tyr His
    50                  55                  60

Tyr Val Val Ser Glu Pro Leu Gly Arg Asn Ser Tyr Lys Glu Arg Tyr
65                  70                  75                  80

Leu Phe Leu Phe Arg Pro Asn Lys Val Ser Val Leu Asp Thr Tyr Gln
                85                  90                  95

Tyr Asp Asp Gly Cys Glu Ser Cys Gly Asn Asp Ser Phe Ser Arg Glu
            100                 105                 110

Pro Ala Val Val Lys Phe Ser Ser His Ser Thr Lys Val Lys Glu Phe
        115                 120                 125

Ala Ile Val Ala Leu His Ser Ala Pro Ser Asp Ala Val Ala Glu Ile
    130                 135                 140

Asn Ser Leu Tyr Asp Val Tyr Leu Asp Val Gln Gln Lys Trp His Leu
145                 150                 155                 160

Asn Asp Val Met Leu Met Gly Asp Phe Asn Ala Asp Cys Ser Tyr Val
                165                 170                 175

Thr Ser Ser Gln Trp Ser Ser Ile Arg Leu Arg Thr Ser Ser Thr Phe
            180                 185                 190

Gln Trp Leu Ile Pro Asp Ser Ala Asp Thr Thr Ala Thr Ser Thr Asn
        195                 200                 205

Cys Ala Tyr Asp Arg Ile Val Val Ala Gly Ser Leu Leu Gln Ser Ser
    210                 215                 220

Val Val Pro Gly Ser Ala Ala Pro Phe Asp Phe Gln Ala Ala Tyr Gly
225                 230                 235                 240

Leu Ser Asn Glu Met Ala Leu Ala Ile Ser Asp His Tyr Pro Val Glu
                245                 250                 255

Val Thr Leu Thr
            260
```

<210> 3
<211> 783

<212> DNA
<213> Artificial Sequence

<220>
<223> Description of Artificial Sequence:encoding a variant comprising three substituted amino acid residues of bovine pancreatic DNase I without signal peptide

<220>
<221> CDS
<222> (1)..(780)

<400> 3

```
ttg aag att gct gct ttc aac att aga act ttc ggt gaa act aaa atg    48
Leu Lys Ile Ala Ala Phe Asn Ile Arg Thr Phe Gly Glu Thr Lys Met
 1               5                  10                  15

tct aac gct act ttg gca tct tac atc gtt aga att gtc aga aga tat    96
Ser Asn Ala Thr Leu Ala Ser Tyr Ile Val Arg Ile Val Arg Arg Tyr
            20                  25                  30

gat atc gtt tta att caa gaa gtt aga gac tct cac ttg gtt gca gtt   144
Asp Ile Val Leu Ile Gln Glu Val Arg Asp Ser His Leu Val Ala Val
        35                  40                  45

ggt aaa ttg tta gac tac ttg aac caa gat gac cca aac act tac cac   192
Gly Lys Leu Leu Asp Tyr Leu Asn Gln Asp Asp Pro Asn Thr Tyr His
    50                  55                  60

tac gtt gtt tct gaa cca ttg ggt aga aac tct tac aaa gaa aga tac   240
Tyr Val Val Ser Glu Pro Leu Gly Arg Asn Ser Tyr Lys Glu Arg Tyr
65                  70                  75                  80

tta ttc ttg ttc aga cca aac aaa gtt tca gtt ttg gat act tac caa   288
Leu Phe Leu Phe Arg Pro Asn Lys Val Ser Val Leu Asp Thr Tyr Gln
                85                  90                  95

tac gac gac ggt tgc gaa tct tgt ggt aac gat tct ttc tcc aga gaa   336
Tyr Asp Asp Gly Cys Glu Ser Cys Gly Asn Asp Ser Phe Ser Arg Glu
            100                 105                 110

cct gct gtt gtt gac ttc tca tca cac tct acc aag gtt aaa gag ttc   384
Pro Ala Val Val Asp Phe Ser Ser His Ser Thr Lys Val Lys Glu Phe
        115                 120                 125

gct atc gtt gct ttg cat tct gct cct tct gac gct gtt gct gaa att   432
Ala Ile Val Ala Leu His Ser Ala Pro Ser Asp Ala Val Ala Glu Ile
    130                 135                 140

aac tct ttg tac gac gtt tac tta gat gtt caa cag aaa tgg cac ttg   480
Asn Ser Leu Tyr Asp Val Tyr Leu Asp Val Gln Gln Lys Trp His Leu
145                 150                 155                 160

aac gac gtc atg ttg atg ggt gac ttt aac gct gat tgc tct tat gtt   528
Asn Asp Val Met Leu Met Gly Asp Phe Asn Ala Asp Cys Ser Tyr Val
                165                 170                 175

act tct tct caa tgg tct tca att gct ttg cac aca tct tca act ttc   576
Thr Ser Ser Gln Trp Ser Ser Ile Ala Leu His Thr Ser Ser Thr Phe
            180                 185                 190

caa tgg tta att cct gat tcc gct gat acc act gct act agt acc aac   624
Gln Trp Leu Ile Pro Asp Ser Ala Asp Thr Thr Ala Thr Ser Thr Asn
        195                 200                 205

tgt gct tac gat aga atc gtt gtt gct gga tca tta ttg caa tct tct   672
Cys Ala Tyr Asp Arg Ile Val Val Ala Gly Ser Leu Leu Gln Ser Ser
    210                 215                 220
```

```
gtt gtc cca ggt tca gcg gcc cct ttc gat ttc caa gct gca tat ggt    720
Val Val Pro Gly Ser Ala Ala Pro Phe Asp Phe Gln Ala Ala Tyr Gly
225                 230                 235                 240

ttg tct aat gaa atg gct tta gcc att tct gat cac tac cca gtt gaa    768
Leu Ser Asn Glu Met Ala Leu Ala Ile Ser Asp His Tyr Pro Val Glu
                245                 250                 255

gtc aca ttg aca taa                                                783
Val Thr Leu Thr
            260
```

<210> 4
<211> 260
<212> PRT
<213> Artificial Sequence
<223> Description of Artificial Sequence:encoding a variant comprising three substituted amino acid residues of bovine pancreatic DNase I without signal peptide

<400> 4

Leu Lys Ile Ala Ala Phe Asn Ile Arg Thr Phe Gly Glu Thr Lys Met
1               5                   10                  15

Ser Asn Ala Thr Leu Ala Ser Tyr Ile Val Arg Ile Val Arg Arg Tyr
            20                  25                  30

Asp Ile Val Leu Ile Gln Glu Val Arg Asp Ser His Leu Val Ala Val
        35                  40                  45

Gly Lys Leu Leu Asp Tyr Leu Asn Gln Asp Asp Pro Asn Thr Tyr His
    50                  55                  60

Tyr Val Val Ser Glu Pro Leu Gly Arg Asn Ser Tyr Lys Glu Arg Tyr
65                  70                  75                  80

Leu Phe Leu Phe Arg Pro Asn Lys Val Ser Val Leu Asp Thr Tyr Gln
                85                  90                  95

Tyr Asp Asp Gly Cys Glu Ser Cys Gly Asn Asp Ser Phe Ser Arg Glu
            100                 105                 110

Pro Ala Val Val Asp Phe Ser Ser His Ser Thr Lys Val Lys Glu Phe
        115                 120                 125

Ala Ile Val Ala Leu His Ser Ala Pro Ser Asp Ala Val Ala Glu Ile
    130                 135                 140

Asn Ser Leu Tyr Asp Val Tyr Leu Asp Val Gln Gln Lys Trp His Leu
145                 150                 155                 160

Asn Asp Val Met Leu Met Gly Asp Phe Asn Ala Asp Cys Ser Tyr Val
                165                 170                 175

Thr Ser Ser Gln Trp Ser Ser Ile Ala Leu His Thr Ser Ser Thr Phe
            180                 185                 190

Gln Trp Leu Ile Pro Asp Ser Ala Asp Thr Thr Ala Thr Ser Thr Asn
        195                 200                 205

Cys Ala Tyr Asp Arg Ile Val Val Ala Gly Ser Leu Leu Gln Ser Ser
    210                 215                 220

Val Val Pro Gly Ser Ala Ala Pro Phe Asp Phe Gln Ala Ala Tyr Gly
225                 230                 235                 240

Leu Ser Asn Glu Met Ala Leu Ala Ile Ser Asp His Tyr Pro Val Glu
                245                 250                 255

Val Thr Leu Thr
            260

<210> 5
<211> 66
<212> DNA
<213> Artificial Sequence

<220>
<223> Description of Artificial Sequence:sequence encoding the signal peptide sequence of the native bovine pancreatic DNase I pre-protein

<400> 5

```
atgagaggta ctagattgat gggtttgtta ttagctttgg ctggtttatt acaattaggt 60
ttgtct                                                            66
```

<210> 6
<211> 78
<212> DNA
<213> Artificial Sequence

<220>
<223> Description of Artificial Sequence:encoding the signal peptide sequence of the native bovine pancreatic DNase I pre-protein and an additional signal peptidase cleavage site

<400> 6

```
atgagaggta ctagattgat gggtttgtta ttagctttgg ctggtttatt acaattaggt 60
ttgtctctcg agaagaga                                                78
```

<210> 7
<211> 255
<212> DNA
<213> Artificial Sequence

<220>
<223> Description of Artificial Sequence:encoding the Saccharomyces cerevisiae a-factor signal peptide sequence and an additional signal peptidase cleavage site

<400> 7

```
atgagatttc cttcaatttt tactgctgtt ttattcgcag catcctccgc attagctgct 60
ccagtcaaca ctacaacaga agatgaaacg gcacaaattc cggctgaagc tgtcatcggt 120
tactcagatt tagaagggga tttcgatgtt gctgttttgc cattttccaa cagcacaaat 180
aacgggttat tgtttataaa tactactatt gccagcattg ctgctaaaga agaaggggta 240
tctctcgaga agaga                                                   255
```

<210> 8
<211> 282
<212> PRT
<213> Artificial Sequence

<220>
<223> Description of Artificial Sequence:A variant of bovine pancreatic DNase I

<400> 8

Met Arg Gly Thr Arg Leu Met Gly Leu Leu Leu Ala Leu Ala Gly Leu
1 5 10 15

Leu Gln Leu Gly Leu Ser Leu Lys Ile Ala Ala Phe Asn Ile Arg Thr
20 25 30

Phe Gly Glu Thr Lys Met Ser Asn Ala Thr Leu Ala Ser Tyr Ile Val
35 40 45

Arg Ile Val Arg Arg Tyr Asp Ile Val Leu Ile Gln Glu Val Arg Asp
50 55 60

Ser His Leu Val Ala Val Gly Lys Leu Leu Asp Tyr Leu Asn Gln Asp
65 70 75 80

Asp Pro Asn Thr Tyr His Tyr Val Val Ser Glu Pro Leu Gly Arg Asn
85 90 95

Ser Tyr Lys Glu Arg Tyr Leu Phe Leu Phe Arg Pro Asn Lys Val Ser
100 105 110

Val Leu Asp Thr Tyr Gln Tyr Asp Asp Gly Cys Glu Ser Cys Gly Asn
115 120 125

Asp Ser Phe Ser Arg Glu Pro Ala Val Val Asp Phe Ser Ser His Ser
130 135 140

Thr Lys Val Lys Glu Phe Ala Ile Val Ala Leu His Ser Ala Pro Ser
145 150 155 160

Asp Ala Val Ala Glu Ile Asn Ser Leu Tyr Asp Val Tyr Leu Asp Val
165 170 175

Gln Gln Lys Trp His Leu Asn Asp Val Met Leu Met Gly Asp Phe Asn
180 185 190

Ala Asp Cys Ser Tyr Val Thr Ser Leu Ser Asn Glu Met Ala Leu Ala
195 200 205

Ile Ser Ser Gln Trp Ser Ser Ile Ala Leu Ala Thr Ser Ser Thr Phe
210 215 220

Gln Trp Leu Ile Pro Asp Ser Ala Asp Thr Thr Ala Thr Ser Thr Asn
225 230 235 240

Cys Ala Tyr Asp Arg Ile Val Val Ala Gly Ser Leu Leu Gln Ser Ser
245 250 255

Val Val Pro Gly Ser Ala Ala Pro Phe Asp Phe Gln Ala Ala Tyr Gly
260 265 270

Asp His Tyr Pro Val Glu Val Thr Leu Thr
275 280

<210> 9

<211> 286
<212> PRT
<213> Artificial Sequence

<220>
<223> Description of Artificial Sequence:Variant of bovine pancreatic DNase I

<400> 9

```
Met Arg Gly Thr Arg Leu Met Gly Leu Leu Leu Ala Leu Ala Gly Leu
 1               5                  10                  15

Leu Gln Leu Gly Leu Ser Leu Glu Lys Arg Leu Lys Ile Ala Ala Phe
            20                  25                  30

Asn Ile Arg Thr Phe Gly Glu Thr Lys Met Ser Asn Ala Thr Leu Ala
        35                  40                  45

Ser Tyr Ile Val Arg Ile Val Arg Arg Tyr Asp Ile Val Leu Ile Gln
    50                  55                  60

Glu Val Arg Asp Ser His Leu Val Ala Val Gly Lys Leu Leu Asp Tyr
65                  70                  75                  80

Leu Asn Gln Asp Asp Pro Asn Thr Tyr His Tyr Val Val Ser Glu Pro
                85                  90                  95

Leu Gly Arg Asn Ser Tyr Lys Glu Arg Tyr Leu Phe Leu Phe Arg Pro
            100                 105                 110

Asn Lys Val Ser Val Leu Asp Thr Tyr Gln Tyr Asp Asp Gly Cys Glu
        115                 120                 125

Ser Cys Gly Asn Asp Ser Phe Ser Arg Glu Pro Ala Val Val Asp Phe
    130                 135                 140

Ser Ser His Ser Thr Lys Val Lys Glu Phe Ala Ile Val Ala Leu His
145                 150                 155                 160

Ser Ala Pro Ser Asp Ala Val Ala Glu Ile Asn Ser Leu Tyr Asp Val
                165                 170                 175

Tyr Leu Asp Val Gln Gln Lys Trp His Leu Asn Asp Val Met Leu Met
            180                 185                 190
```

```
Gly Asp Phe Asn Ala Asp Cys Ser Tyr Val Thr Ser Leu Ser Asn Glu
        195                 200                 205

Met Ala Leu Ala Ile Ser Ser Gln Trp Ser Ser Ile Ala Leu Ala Thr
    210                 215                 220

Ser Ser Thr Phe Gln Trp Leu Ile Pro Asp Ser Ala Asp Thr Thr Ala
225                 230                 235                 240

Thr Ser Thr Asn Cys Ala Tyr Asp Arg Ile Val Val Ala Gly Ser Leu
                245                 250                 255

Leu Gln Ser Ser Val Val Pro Gly Ser Ala Ala Pro Phe Asp Phe Gln
            260                 265                 270

Ala Ala Tyr Gly Asp His Tyr Pro Val Glu Val Thr Leu Thr
        275                 280                 285
```

<210> 10
<211> 351
<212> PRT
<213> Artificial Sequence

<220>
<223> Description of Artificial Sequence:Variant of bovine pancreatic DNase I

<400> 10

```
Met Arg Phe Pro Ser Ile Phe Thr Ala Val Leu Phe Ala Ala Ser Ser
 1               5                  10                  15

Ala Leu Ala Ala Pro Val Asn Thr Thr Thr Glu Asp Glu Thr Ala Gln
            20                  25                  30

Ile Pro Ala Glu Ala Val Ile Gly Tyr Ser Asp Leu Glu Gly Asp Phe
        35                  40                  45

Asp Val Ala Val Leu Pro Phe Ser Asn Ser Thr Asn Asn Gly Leu Leu
    50                  55                  60

Phe Ile Asn Thr Thr Ile Ala Ser Ile Ala Ala Lys Glu Glu Gly Val
65                  70                  75                      80

Ser Leu Glu Lys Arg Leu Ser Leu Glu Lys Arg Leu Lys Ile Ala Ala
                85                  90                  95

Phe Asn Ile Arg Thr Phe Gly Glu Thr Lys Met Ser Asn Ala Thr Leu
            100                 105                 110

Ala Ser Tyr Ile Val Arg Ile Val Arg Arg Tyr Asp Ile Val Leu Ile
        115                 120                 125

Gln Glu Val Arg Asp Ser His Leu Val Ala Val Gly Lys Leu Leu Asp
    130                 135                 140

Tyr Leu Asn Gln Asp Asp Pro Asn Thr Tyr His Tyr Val Val Ser Glu
145                 150                 155                 160
```

```
Pro Leu Gly Arg Asn Ser Tyr Lys Glu Arg Tyr Leu Phe Leu Phe Arg
                165                 170                 175

Pro Asn Lys Val Ser Val Leu Asp Thr Tyr Gln Tyr Asp Asp Gly Cys
            180                 185                 190

Glu Ser Cys Gly Asn Asp Ser Phe Ser Arg Glu Pro Ala Val Val Asp
        195                 200                 205

Phe Ser Ser His Ser Thr Lys Val Lys Glu Phe Ala Ile Val Ala Leu
    210                 215                 220

His Ser Ala Pro Ser Asp Ala Val Ala Glu Ile Asn Ser Leu Tyr Asp
225                 230                 235                 240

Val Tyr Leu Asp Val Gln Gln Lys Trp His Leu Asn Asp Val Met Leu
                245                 250                 255

Met Gly Asp Phe Asn Ala Asp Cys Ser Tyr Val Thr Ser Leu Ser Asn
            260                 265                 270

Glu Met Ala Leu Ala Ile Ser Ser Gln Trp Ser Ser Ile Ala Leu Ala
        275                 280                 285

Thr Ser Ser Thr Phe Gln Trp Leu Ile Pro Asp Ser Ala Asp Thr Thr
    290                 295                 300

Ala Thr Ser Thr Asn Cys Ala Tyr Asp Arg Ile Val Val Ala Gly Ser
305                 310                 315                 320

Leu Leu Gln Ser Ser Val Val Pro Gly Ser Ala Ala Pro Phe Asp Phe
                325                 330                 335

Gln Ala Ala Tyr Gly Asp His Tyr Pro Val Glu Val Thr Leu Thr
            340                 345                 350
```

<210> 11
<211> 938
<212> DNA
<213> Pichia pastoris

<220>
<223> Nucleotide sequence of the Pichia pastoris AOX1 promoter

<400> 11

agatctaaca tccaaagacg aaaggttgaa tgaaaccttt ttgccatccg acatccacag 60
gtccattctc acacataagt gccaaacgca acaggagggg atacactagc agcagaccgt 120
tgcaaacgca ggacctccac tcctcttctc ctcaacaccc acttttgcca tcgaaaaacc 180
agcccagtta ttgggcttga ttggagctcg ctcattccaa ttccttctat taggctacta 240
acaccatgac tttattagcc tgtctatcct ggccccccctg gcgaggttca tgtttgttta 300
tttccgaatg caacaagctc cgcattacac ccgaacatca ctccagatga gggctttctg 360
agtgtggggt caaatagttt catgttcccc aaatggccca aaactgacag tttaaacgct 420
gtcttggaac ctaatatgac aaaagcgtga tctcatccaa gatgaactaa gtttggttcg 480
ttgaaatgct aacggccagt tggtcaaaaa gaaacttcca aaagtcggca taccgtttgt 540
cttgtttggt attgattgac gaatgctcaa aaataatctc attaatgctt agcgcagtct 600
ctctatcgct tctgaacccc ggtgcacctg tgccgaaacg caaatgggga aacacccgct 660

ttttggatga ttatgcattg tctccacatt gtatgcttcc aagattctgg tgggaatact 720
gctgatagcc taacgttcat gatcaaaatt taactgttct aacccctact tgacagcaat 780
atataaacag aaggaagctg ccctgtctta aacctttttt tttatcatca ttattagctt 840
actttcataa ttgcgactgg ttccaattga caagcttttg attttaacga cttttaacga 900
caacttgaga agatcaaaaa acaactaatt attcgaaa 938

<210> 12
<211> 39
<212> DNA
<213> Artificial Sequence

<220>
<223> Description of Artificial Sequence:primer

<400> 12
gtatctctcg agaaaagatt gaagattgct gctttcaac 39

<210> 13
<211> 36
<212> DNA
<213> Artificial Sequence

<220>
<223> Description of Artificial Sequence:primer

<400> 13
ctggcggccg cttatgtcaa tgtgacttca actggg 36

<210> 14
<211> 23
<212> DNA
<213> Artificial Sequence

<220>
<223> Description of Artificial Sequence:primer

<400> 14
ctttaacgct gatgcctctt atg 23

<210> 15
<211> 25
<212> DNA

<213> Artificial Sequence

<220>
<223> Description of Artificial Sequence:primer

<400> 15
cataagaggc atcagcgtta aagtc 25

<210> 16
<211> 33
<212> DNA
<213> Artificial Sequence

<220>
<223> Description of Artificial Sequence:primer

<400> 16
cgacgacggt gccgaatctt gtggtaacga ttc 33

<210> 17
<211> 33
<212> DNA
<213> Artificial Sequence

<220>
<223> Description of Artificial Sequence:primer

<400> 17
gaatcgttac cacaagattc ggcaccgtcg tcg 33

<210> 18
<211> 33
<212> DNA
<213> Artificial Sequence

<220>
<223> Description of Artificial Sequence:primer

<400> 18
cgacgacggt tgcgaatctg ctggtaacga ttc 33

<210> 19
<211> 33
<212> DNA
<213> Artificial Sequence

<220>
<223> Description of Artificial Sequence:primer

<400> 19
gaatcgttac cagcagattc gcaaccgtcg tcg 33

<210> 20
<211> 29
<212> DNA
<213> Artificial Sequence

<220>

<223> Description of Artificial Sequence:primer

<400> 20
cctgctgttg ttgacttctc atcacactc 29

<210> 21
<211> 29
<212> DNA
<213> Artificial Sequence

<220>
<223> Description of Artificial Sequence:primer

<400> 21
gagtgtgatg agaagtcaac aacagcagg 29

<210> 22
<211> 37
<212> DNA
<213> Artificial Sequence

<220>
<223> Description of Artificial Sequence:primer

<400> 22
ctcaatggtc ttcaattcac ttgagaacat cttcaac 37

<210> 23
<211> 37
<212> DNA
<213> Artificial Sequence

<220>
<223> Description of Artificial Sequence:primer

<400> 23
gttgaagatg ttctcaagtg aattgaagac cattgag 37

<210> 24
<211> 37
<212> DNA
<213> Artificial Sequence

<220>
<223> Description of Artificial Sequence:primer

<400> 24
ctcaatggtc ttcaattgca ttgagaacat cttcaac 37

<210> 25
<211> 37
<212> DNA
<213> Artificial Sequence

<220>
<223> Description of Artificial Sequence:primer

<400> 25

gttgaagatg ttctcaatgc aattgaagac cattgag 37

<210> 26
<211> 36
<212> DNA
<213> Artificial Sequence

<220>
<223> Description of Artificial Sequence:primer

<400> 26
ggtcttcaat tagattgcac acatcttcaa ctttcc 36

<210> 27
<211> 36
<212> DNA
<213> Artificial Sequence

<220>
<223> Description of Artificial Sequence:primer

<400> 27
ggaaagttga agatgtgtgc aatctaattg aagacc 36

<210> 28
<211> 36
<212> DNA
<213> Artificial Sequence

<220>
<223> Description of Artificial Sequence:primer

<400> 28
ggtcttcaat tagattggca acatcttcaa ctttcc 36

<210> 29
<211> 36
<212> DNA
<213> Artificial Sequence

<220>
<223> Description of Artificial Sequence:primer

<400> 29
ggaaagttga agatgttgcc aatctaattg aagacc 36

<210> 30
<211> 39
<212> DNA
<213> Artificial Sequence

<220>
<223> Description of Artificial Sequence:primer

<400> 30
gtatctctcg agaaaagatt gaagtctgct gctttcaac 39

<210> 31

<211> 30
<212> DNA
<213> Artificial Sequence

<220>
<223> Description of Artificial Sequence:primer

<400> 31
caaagaaaga tacttaaact tgttcagacc 30

<210> 32
<211> 30
<212> DNA
<213> Artificial Sequence

<220>
<223> Description of Artificial Sequence:primer

<400> 32
ggtctgaaca agtttaagta tctttctttg 30

<210> 33
<211> 28
<212> DNA
<213> Artificial Sequence

<220>
<223> Description of Artificial Sequence:primer

<400> 33
ccaaggttaa agagaacgct atcgttgc 28

<210> 34
<211> 28
<212> DNA
<213> Artificial Sequence

<220>
<223> Description of Artificial Sequence:primer

<400> 34
gcaacgatag cgttctcttt aaccttgg 28

<210> 35
<211> 33
<212> DNA
<213> Artificial Sequence

<220>
<223> Description of Artificial Sequence:primer

<400> 35
cgacgacggt gccgaatctg ctggtaacga ttc 33

<210> 36
<211> 33
<212> DNA
<213> Artificial Sequence

<220>
<223> Description of Artificial Sequence:primer

<400> 36
gaatcgttac cagcagattc ggcaccgtcg tcg 33

<210> 37
<211> 42
<212> DNA
<213> Artificial Sequence

<220>
<223> Description of Artificial Sequence:primer

<400> 37
ctcaatggtc ttcaattgca ttgcacacat cttcaacttt cc 42

<210> 38
<211> 42
<212> DNA
<213> Artificial Sequence

<220>
<223> Description of Artificial Sequence:primer

<400> 38
ggaaagttga agatgtgtgc aatgcaattg aagaccattg ag 42

<210> 39
<211> 73
<212> DNA
<213> Artificial Sequence

<220>
<223> Description of Artificial Sequence:primer

<400> 39

cgaaaaatga gaggtactag attgatgggt ttgttattag ctttggctgg tttattacaa 60
ttaggtttgt ctc 73

<210> 40
<211> 75
<212> DNA
<213> Artificial Sequence

<220>
<223> Description of Artificial Sequence:primer

<400> 40

tcgagagaca aacctaattg taataaacca gccaaagcta ataacaaacc catcaatcta 60
gtacctctca ttttt 75

SEQUENCE LISTING

<110> Roche Diagnostics GmbH F. Hoffmann-La Roche AG

<120> Heat-labile desoxyribonuclease I variants

<130> 21551 EP

<140>
<141>

<160> 40

<170> Patent In Ver. 2.1

<210> 1
<211> 783
<212> DNA
<213> Artificial Sequence

<220>
<223> Description of Artificial Sequence:sequence encoding the wild-type bovine pancreatic DNase I without signal peptide

<220>
<221> CDS
<222> (1)..(780)

<400> 1

```
ttg aag att gct gct ttc aac att aga act ttc ggt gaa act aaa atg    48
Leu Lys Ile Ala Ala Phe Asn Ile Arg Thr Phe Gly Glu Thr Lys Met
 1               5                  10                  15

tct aac gct act ttg gca tct tac atc gtt aga att gtc aga aga tat    96
Ser Asn Ala Thr Leu Ala Ser Tyr Ile Val Arg Ile Val Arg Arg Tyr
            20                  25                  30

gat atc gtt tta att caa gaa gtt aga gac tct cac ttg gtt gca gtt   144
Asp Ile Val Leu Ile Gln Glu Val Arg Asp Ser His Leu Val Ala Val
        35                  40                  45

ggt aaa ttg tta gac tac ttg aac caa gat gac cca aac act tac cac   192
Gly Lys Leu Leu Asp Tyr Leu Asn Gln Asp Asp Pro Asn Thr Tyr His
    50                  55                  60

tac gtt gtt tct gaa cca ttg ggt aga aac tct tac aaa gaa aga tac   240
Tyr Val Val Ser Glu Pro Leu Gly Arg Asn Ser Tyr Lys Glu Arg Tyr
65                  70                  75                  80

tta ttc ttg ttc aga cca aac aaa gtt tca gtt ttg gat act tac caa   288
Leu Phe Leu Phe Arg Pro Asn Lys Val Ser Val Leu Asp Thr Tyr Gln
                85                  90                  95

tac gac gac ggt tgc gaa tct tgt ggt aac gat tct ttc tcc aga gaa   336
Tyr Asp Asp Gly Cys Glu Ser Cys Gly Asn Asp Ser Phe Ser Arg Glu
            100                 105                 110
```

```
cct gct gtt gtt aaa ttc tca tca cac tct acc aag gtt aaa gag ttc    384
Pro Ala Val Val Lys Phe Ser Ser His Ser Thr Lys Val Lys Glu Phe
        115                 120                 125

gct atc gtt gct ttg cat tct gct cct tct gac gct gtt gct gaa att    432
Ala Ile Val Ala Leu His Ser Ala Pro Ser Asp Ala Val Ala Glu Ile
    130                 135                 140

aac tct ttg tac gac gtt tac tta gat gtt caa cag aaa tgg cac ttg    480
Asn Ser Leu Tyr Asp Val Tyr Leu Asp Val Gln Gln Lys Trp His Leu
145                 150                 155                 160

aac gac gtc atg ttg atg ggt gac ttt aac gct gat tgc tct tat gtt    528
Asn Asp Val Met Leu Met Gly Asp Phe Asn Ala Asp Cys Ser Tyr Val
                165                 170                 175

act tct tct caa tgg tct tca att aga ttg aga aca tct tca act ttc    576
Thr Ser Ser Gln Trp Ser Ser Ile Arg Leu Arg Thr Ser Ser Thr Phe
            180                 185                 190

caa tgg tta att cct gat tcc gct gat acc act gct act agt acc aac    624
Gln Trp Leu Ile Pro Asp Ser Ala Asp Thr Thr Ala Thr Ser Thr Asn
        195                 200                 205

tgt gct tac gat aga atc gtt gtt gct gga tca tta ttg caa tct tct    672
Cys Ala Tyr Asp Arg Ile Val Val Ala Gly Ser Leu Leu Gln Ser Ser
    210                 215                 220

gtt gtc cca ggt tca gcg gcc cct ttc gat ttc caa gct gca tat ggt    720
Val Val Pro Gly Ser Ala Ala Pro Phe Asp Phe Gln Ala Ala Tyr Gly
225                 230                 235                 240

ttg tct aat gaa atg gct tta gcc att tct gat cac tac cca gtt gaa    768
Leu Ser Asn Glu Met Ala Leu Ala Ile Ser Asp His Tyr Pro Val Glu
                245                 250                 255

gtc aca ttg aca taa                                                783
Val Thr Leu Thr
            260
```

<210> 2
<211> 260
<212> PRT
<213> Artificial Sequence
<223> Description of Artificial Sequence:sequence encoding the wild-type bovine pancreatic DNase I without signal peptide

<400> 2

```
Leu Lys Ile Ala Ala Phe Asn Ile Arg Thr Phe Gly Glu Thr Lys Met
 1               5                  10                  15

Ser Asn Ala Thr Leu Ala Ser Tyr Ile Val Arg Ile Val Arg Arg Tyr
            20                  25                  30

Asp Ile Val Leu Ile Gln Glu Val Arg Asp Ser His Leu Val Ala Val
        35                  40                  45

Gly Lys Leu Leu Asp Tyr Leu Asn Gln Asp Asp Pro Asn Thr Tyr His
    50                  55                  60

Tyr Val Val Ser Glu Pro Leu Gly Arg Asn Ser Tyr Lys Glu Arg Tyr
65                  70                  75                  80

Leu Phe Leu Phe Arg Pro Asn Lys Val Ser Val Leu Asp Thr Tyr Gln
                85                  90                  95

Tyr Asp Asp Gly Cys Glu Ser Cys Gly Asn Asp Ser Phe Ser Arg Glu
            100                 105                 110

Pro Ala Val Val Lys Phe Ser Ser His Ser Thr Lys Val Lys Glu Phe
        115                 120                 125

Ala Ile Val Ala Leu His Ser Ala Pro Ser Asp Ala Val Ala Glu Ile
    130                 135                 140

Asn Ser Leu Tyr Asp Val Tyr Leu Asp Val Gln Gln Lys Trp His Leu
145                 150                 155                 160

Asn Asp Val Met Leu Met Gly Asp Phe Asn Ala Asp Cys Ser Tyr Val
                165                 170                 175

Thr Ser Ser Gln Trp Ser Ser Ile Arg Leu Arg Thr Ser Ser Thr Phe
            180                 185                 190

Gln Trp Leu Ile Pro Asp Ser Ala Asp Thr Thr Ala Thr Ser Thr Asn
        195                 200                 205

Cys Ala Tyr Asp Arg Ile Val Val Ala Gly Ser Leu Leu Gln Ser Ser
    210                 215                 220

Val Val Pro Gly Ser Ala Ala Pro Phe Asp Phe Gln Ala Ala Tyr Gly
225                 230                 235                 240

Leu Ser Asn Glu Met Ala Leu Ala Ile Ser Asp His Tyr Pro Val Glu
                245                 250                 255

Val Thr Leu Thr
            260
```

<210> 3
<211> 783
<212> DNA
<213> Artificial Sequence

<220>
<223> Description of Artificial Sequence:encoding a variant comprising three substituted amino acid residues of bovine pancreatic DNase I without signal peptide

<220>
<221> CDS
<222> (1)..(780)

<400> 3

```
ttg aag att gct gct ttc aac att aga act ttc ggt gaa act aaa atg    48
Leu Lys Ile Ala Ala Phe Asn Ile Arg Thr Phe Gly Glu Thr Lys Met
 1               5                  10                  15

tct aac gct act ttg gca tct tac atc gtt aga att gtc aga aga tat    96
Ser Asn Ala Thr Leu Ala Ser Tyr Ile Val Arg Ile Val Arg Arg Tyr
            20                  25                  30

gat atc gtt tta att caa gaa gtt aga gac tct cac ttg gtt gca gtt   144
Asp Ile Val Leu Ile Gln Glu Val Arg Asp Ser His Leu Val Ala Val
        35                  40                  45

ggt aaa ttg tta gac tac ttg aac caa gat gac cca aac act tac cac   192
Gly Lys Leu Leu Asp Tyr Leu Asn Gln Asp Asp Pro Asn Thr Tyr His
    50                  55                  60

tac gtt gtt tct gaa cca ttg ggt aga aac tct tac aaa gaa aga tac   240
Tyr Val Val Ser Glu Pro Leu Gly Arg Asn Ser Tyr Lys Glu Arg Tyr
65                  70                  75                  80

tta ttc ttg ttc aga cca aac aaa gtt tca gtt ttg gat act tac caa   288
Leu Phe Leu Phe Arg Pro Asn Lys Val Ser Val Leu Asp Thr Tyr Gln
                85                  90                  95

tac gac gac ggt tgc gaa tct tgt ggt aac gat tct ttc tcc aga gaa   336
Tyr Asp Asp Gly Cys Glu Ser Cys Gly Asn Asp Ser Phe Ser Arg Glu
            100                 105                 110

cct gct gtt gtt gac ttc tca tca cac tct acc aag gtt aaa gag ttc   384
Pro Ala Val Val Asp Phe Ser Ser His Ser Thr Lys Val Lys Glu Phe
        115                 120                 125

gct atc gtt gct ttg cat tct gct cct tct gac gct gtt gct gaa att   432
Ala Ile Val Ala Leu His Ser Ala Pro Ser Asp Ala Val Ala Glu Ile
    130                 135                 140

aac tct ttg tac gac gtt tac tta gat gtt caa cag aaa tgg cac ttg   480
Asn Ser Leu Tyr Asp Val Tyr Leu Asp Val Gln Gln Lys Trp His Leu
145                 150                 155                 160

aac gac gtc atg ttg atg ggt gac ttt aac gct gat tgc tct tat gtt   528
Asn Asp Val Met Leu Met Gly Asp Phe Asn Ala Asp Cys Ser Tyr Val
                165                 170                 175

act tct tct caa tgg tct tca att gct ttg cac aca tct tca act ttc   576
Thr Ser Ser Gln Trp Ser Ser Ile Ala Leu His Thr Ser Ser Thr Phe
            180                 185                 190

caa tgg tta att cct gat tcc gct gat acc act gct act agt acc aac   624
Gln Trp Leu Ile Pro Asp Ser Ala Asp Thr Thr Ala Thr Ser Thr Asn
        195                 200                 205

tgt gct tac gat aga atc gtt gtt gct gga tca tta ttg caa tct tct   672
Cys Ala Tyr Asp Arg Ile Val Val Ala Gly Ser Leu Leu Gln Ser Ser
    210                 215                 220
```

```
gtt gtc cca ggt tca gcg gcc cct ttc gat ttc caa gct gca tat ggt    720
Val Val Pro Gly Ser Ala Ala Pro Phe Asp Phe Gln Ala Ala Tyr Gly
225                 230                 235                 240

ttg tct aat gaa atg gct tta gcc att tct gat cac tac cca gtt gaa    768
Leu Ser Asn Glu Met Ala Leu Ala Ile Ser Asp His Tyr Pro Val Glu
                245                 250                 255

gtc aca ttg aca taa                                                783
Val Thr Leu Thr
            260
```

<210> 4
<211> 260
<212> PRT
<213> Artificial Sequence
<223> Description of Artificial Sequence:encoding a variant comprising three substituted amino acid residues of bovine pancreatic DNase I without signal peptide

<400> 4

```
Leu Lys Ile Ala Ala Phe Asn Ile Arg Thr Phe Gly Glu Thr Lys Met
 1               5                   10                  15

Ser Asn Ala Thr Leu Ala Ser Tyr Ile Val Arg Ile Val Arg Arg Tyr
            20                  25                  30

Asp Ile Val Leu Ile Gln Glu Val Arg Asp Ser His Leu Val Ala Val
        35                  40                  45

Gly Lys Leu Leu Asp Tyr Leu Asn Gln Asp Asp Pro Asn Thr Tyr His
    50                  55                  60

Tyr Val Val Ser Glu Pro Leu Gly Arg Asn Ser Tyr Lys Glu Arg Tyr
65                  70                  75                  80

Leu Phe Leu Phe Arg Pro Asn Lys Val Ser Val Leu Asp Thr Tyr Gln
                85                  90                  95

Tyr Asp Asp Gly Cys Glu Ser Cys Gly Asn Asp Ser Phe Ser Arg Glu
            100                 105                 110

Pro Ala Val Val Asp Phe Ser Ser His Ser Thr Lys Val Lys Glu Phe
        115                 120                 125

Ala Ile Val Ala Leu His Ser Ala Pro Ser Asp Ala Val Ala Glu Ile
    130                 135                 140

Asn Ser Leu Tyr Asp Val Tyr Leu Asp Val Gln Gln Lys Trp His Leu
145                 150                 155                 160

Asn Asp Val Met Leu Met Gly Asp Phe Asn Ala Asp Cys Ser Tyr Val
                165                 170                 175

Thr Ser Ser Gln Trp Ser Ser Ile Ala Leu His Thr Ser Ser Thr Phe
            180                 185                 190

Gln Trp Leu Ile Pro Asp Ser Ala Asp Thr Thr Ala Thr Ser Thr Asn
        195                 200                 205

Cys Ala Tyr Asp Arg Ile Val Val Ala Gly Ser Leu Leu Gln Ser Ser
    210                 215                 220

Val Val Pro Gly Ser Ala Ala Pro Phe Asp Phe Gln Ala Ala Tyr Gly
225                 230                 235                 240

Leu Ser Asn Glu Met Ala Leu Ala Ile Ser Asp His Tyr Pro Val Glu
                245                 250                 255

Val Thr Leu Thr
            260
```

<210> 5
<211> 66
<212> DNA

<213> Artificial Sequence

<220>
<223> Description of Artificial Sequence:sequence encoding the signal peptide sequence of the native bovine pancreatic DNase I pre-protein

<400> 5

```
atgagaggta ctagattgat gggtttgtta ttagctttgg ctggtttatt acaattaggt 60
ttgtct                                                            66
```

<210> 6
<211> 78
<212> DNA
<213> Artificial Sequence

<220>
<223> Description of Artificial Sequence:encoding the signal peptide sequence of the native bovine pancreatic DNase I pre-protein and an additional signal peptidase cleavage site

<400> 6

```
atgagaggta ctagattgat gggtttgtta ttagctttgg ctggtttatt acaattaggt 60
ttgtctctcg agaagaga                                                78
```

<210> 7
<211> 255
<212> DNA
<213> Artificial Sequence

<220>
<223> Description of Artificial Sequence:encoding the Saccharomyces cerevisiae a-factor signal peptide sequence and an additional signal peptidase cleavage site

<400> 7

```
atgagatttc cttcaatttt tactgctgtt ttattcgcag catcctccgc attagctgct 60
ccagtcaaca ctacaacaga agatgaaacg gcacaaattc cggctgaagc tgtcatcggt 120
tactcagatt tagaagggga tttcgatgtt gctgttttgc cattttccaa cagcacaaat 180
aacgggttat tgtttataaa tactactatt gccagcattg ctgctaaaga agaaggggta 240
tctctcgaga agaga                                                  255
```

<210> 8
<211> 282
<212> PRT
<213> Artificial Sequence

<220>
<223> Description of Artificial Sequence:A variant of bovine pancreatic DNase I

<400> 8

```
Met Arg Gly Thr Arg Leu Met Gly Leu Leu Leu Ala Leu Ala Gly Leu
 1               5                  10                  15

Leu Gln Leu Gly Leu Ser Leu Lys Ile Ala Ala Phe Asn Ile Arg Thr
            20                  25                  30

Phe Gly Glu Thr Lys Met Ser Asn Ala Thr Leu Ala Ser Tyr Ile Val
        35                  40                  45

Arg Ile Val Arg Arg Tyr Asp Ile Val Leu Ile Gln Glu Val Arg Asp
    50                  55                  60

Ser His Leu Val Ala Val Gly Lys Leu Leu Asp Tyr Leu Asn Gln Asp
 65                 70                  75                  80

Asp Pro Asn Thr Tyr His Tyr Val Val Ser Glu Pro Leu Gly Arg Asn
                85                  90                  95

Ser Tyr Lys Glu Arg Tyr Leu Phe Leu Phe Arg Pro Asn Lys Val Ser
            100                 105                 110

Val Leu Asp Thr Tyr Gln Tyr Asp Asp Gly Cys Glu Ser Cys Gly Asn
        115                 120                 125

Asp Ser Phe Ser Arg Glu Pro Ala Val Val Asp Phe Ser Ser His Ser
    130                 135                 140

Thr Lys Val Lys Glu Phe Ala Ile Val Ala Leu His Ser Ala Pro Ser
145                 150                 155                 160

Asp Ala Val Ala Glu Ile Asn Ser Leu Tyr Asp Val Tyr Leu Asp Val
                165                 170                 175

Gln Gln Lys Trp His Leu Asn Asp Val Met Leu Met Gly Asp Phe Asn
            180                 185                 190

Ala Asp Cys Ser Tyr Val Thr Ser Leu Ser Asn Glu Met Ala Leu Ala
        195                 200                 205

Ile Ser Ser Gln Trp Ser Ser Ile Ala Leu Ala Thr Ser Ser Thr Phe
    210                 215                 220

Gln Trp Leu Ile Pro Asp Ser Ala Asp Thr Thr Ala Thr Ser Thr Asn
225                 230                 235                 240

Cys Ala Tyr Asp Arg Ile Val Val Ala Gly Ser Leu Leu Gln Ser Ser
                245                 250                 255

Val Val Pro Gly Ser Ala Ala Pro Phe Asp Phe Gln Ala Ala Tyr Gly
            260                 265                 270

Asp His Tyr Pro Val Glu Val Thr Leu Thr
        275                 280
```

<210> 9

<211> 286
<212> PRT
<213> Artificial Sequence

<220>
<223> Description of Artificial Sequence:Variant of bovine pancreatic DNase I

<400> 9

```
Met Arg Gly Thr Arg Leu Met Gly Leu Leu Leu Ala Leu Ala Gly Leu
1               5                   10                  15

Leu Gln Leu Gly Leu Ser Leu Glu Lys Arg Leu Lys Ile Ala Ala Phe
            20                  25                  30

Asn Ile Arg Thr Phe Gly Glu Thr Lys Met Ser Asn Ala Thr Leu Ala
        35                  40                  45

Ser Tyr Ile Val Arg Ile Val Arg Arg Tyr Asp Ile Val Leu Ile Gln
    50                  55                  60

Glu Val Arg Asp Ser His Leu Val Ala Val Gly Lys Leu Leu Asp Tyr
65                  70                  75                  80

Leu Asn Gln Asp Asp Pro Asn Thr Tyr His Tyr Val Val Ser Glu Pro
                85                  90                  95

Leu Gly Arg Asn Ser Tyr Lys Glu Arg Tyr Leu Phe Leu Phe Arg Pro
            100                 105                 110

Asn Lys Val Ser Val Leu Asp Thr Tyr Gln Tyr Asp Asp Gly Cys Glu
        115                 120                 125

Ser Cys Gly Asn Asp Ser Phe Ser Arg Glu Pro Ala Val Val Asp Phe
    130                 135                 140

Ser Ser His Ser Thr Lys Val Lys Glu Phe Ala Ile Val Ala Leu His
145                 150                 155                 160

Ser Ala Pro Ser Asp Ala Val Ala Glu Ile Asn Ser Leu Tyr Asp Val
                165                 170                 175

Tyr Leu Asp Val Gln Gln Lys Trp His Leu Asn Asp Val Met Leu Met
            180                 185                 190
```

Gly Asp Phe Asn Ala Asp Cys Ser Tyr Val Thr Ser Leu Ser Asn Glu
195 200 205

Met Ala Leu Ala Ile Ser Ser Gln Trp Ser Ser Ile Ala Leu Ala Thr
210 215 220

Ser Ser Thr Phe Gln Trp Leu Ile Pro Asp Ser Ala Asp Thr Thr Ala
225 230 235 240

Thr Ser Thr Asn Cys Ala Tyr Asp Arg Ile Val Val Ala Gly Ser Leu
245 250 255

Leu Gln Ser Ser Val Val Pro Gly Ser Ala Ala Pro Phe Asp Phe Gln
260 265 270

Ala Ala Tyr Gly Asp His Tyr Pro Val Glu Val Thr Leu Thr
275 280 285

<210> 10
<211> 351
<212> PRT
<213> Artificial Sequence

<220>
<223> Description of Artificial Sequence:Variant of bovine pancreatic DNase I

<400> 10

```
Met Arg Phe Pro Ser Ile Phe Thr Ala Val Leu Phe Ala Ala Ser Ser
1               5                   10                  15

Ala Leu Ala Ala Pro Val Asn Thr Thr Thr Glu Asp Glu Thr Ala Gln
            20                  25                  30

Ile Pro Ala Glu Ala Val Ile Gly Tyr Ser Asp Leu Glu Gly Asp Phe
        35                  40                  45

Asp Val Ala Val Leu Pro Phe Ser Asn Ser Thr Asn Asn Gly Leu Leu
    50                  55                  60

Phe Ile Asn Thr Thr Ile Ala Ser Ile Ala Ala Lys Glu Glu Gly Val
65                  70                  75                  80

Ser Leu Glu Lys Arg Leu Ser Leu Glu Lys Arg Leu Lys Ile Ala Ala
                85                  90                  95

Phe Asn Ile Arg Thr Phe Gly Glu Thr Lys Met Ser Asn Ala Thr Leu
            100                 105                 110

Ala Ser Tyr Ile Val Arg Ile Val Arg Arg Tyr Asp Ile Val Leu Ile
        115                 120                 125

Gln Glu Val Arg Asp Ser His Leu Val Ala Val Gly Lys Leu Leu Asp
    130                 135                 140

Tyr Leu Asn Gln Asp Asp Pro Asn Thr Tyr His Tyr Val Val Ser Glu
145                 150                 155                 160
```

```
Pro Leu Gly Arg Asn Ser Tyr Lys Glu Arg Tyr Leu Phe Leu Phe Arg
                165                 170                 175

Pro Asn Lys Val Ser Val Leu Asp Thr Tyr Gln Tyr Asp Asp Gly Cys
            180                 185                 190

Glu Ser Cys Gly Asn Asp Ser Phe Ser Arg Glu Pro Ala Val Val Asp
        195                 200                 205

Phe Ser Ser His Ser Thr Lys Val Lys Glu Phe Ala Ile Val Ala Leu
    210                 215                 220

His Ser Ala Pro Ser Asp Ala Val Ala Glu Ile Asn Ser Leu Tyr Asp
225                 230                 235                 240

Val Tyr Leu Asp Val Gln Gln Lys Trp His Leu Asn Asp Val Met Leu
                245                 250                 255

Met Gly Asp Phe Asn Ala Asp Cys Ser Tyr Val Thr Ser Leu Ser Asn
            260                 265                 270

Glu Met Ala Leu Ala Ile Ser Ser Gln Trp Ser Ser Ile Ala Leu Ala
        275                 280                 285

Thr Ser Ser Thr Phe Gln Trp Leu Ile Pro Asp Ser Ala Asp Thr Thr
    290                 295                 300

Ala Thr Ser Thr Asn Cys Ala Tyr Asp Arg Ile Val Val Ala Gly Ser
305                 310                 315                 320

Leu Leu Gln Ser Ser Val Val Pro Gly Ser Ala Ala Pro Phe Asp Phe
                325                 330                 335

Gln Ala Ala Tyr Gly Asp His Tyr Pro Val Glu Val Thr Leu Thr
            340                 345                 350
```

<210> 11
<211> 938
<212> DNA
<213> Pichia pastoris

<220>
<223> Nucleotide sequence of the Pichia pastoris AOX1 promoter

<400> 11

```
agatctaaca tccaaagacg aaaggttgaa tgaaaccttt ttgccatccg acatccacag 60
gtccattctc acacataagt gccaaacgca acaggagggg atacactagc agcagaccgt 120
tgcaaacgca ggacctccac tcctcttctc ctcaacaccc acttttgcca tcgaaaaacc 180
agcccagtta ttgggcttga ttggagctcg ctcattccaa ttccttctat taggctacta 240
acaccatgac tttattagcc tgtctatcct ggcccccctg gcgaggttca tgtttgttta 300
tttccgaatg caacaagctc cgcattacac ccgaacatca ctccagatga gggctttctg 360
agtgtggggt caaatagttt catgttcccc aaatggccca aaactgacag tttaaacgct 420
gtcttggaac ctaatatgac aaaagcgtga tctcatccaa gatgaactaa gtttggttcg 480
ttgaaatgct aacggccagt tggtcaaaaa gaaacttcca aaagtcggca taccgtttgt 540
cttgtttggt attgattgac gaatgctcaa aaataatctc attaatgctt agcgcagtct 600
ctctatcgct tctgaacccc ggtgcacctg tgccgaaacg caaatgggga aacacccgct 660
```

```
ttttggatga ttatgcattg tctccacatt gtatgcttcc aagattctgg tgggaatact 720
gctgatagcc taacgttcat gatcaaaatt taactgttct aacccctact tgacagcaat 780
atataaacag aaggaagctg ccctgtctta aaccttttt tttatcatca ttattagctt 840
actttcataa ttgcgactgg ttccaattga caagcttttg attttaacga cttttaacga 900
caacttgaga agatcaaaaa acaactaatt attcgaaa                         938
```

<210> 12
<211> 39
<212> DNA
<213> Artificial Sequence

<220>
<223> Description of Artificial Sequence:primer

<400> 12
gtatctctcg agaaaagatt gaagattgct gctttcaac 39

<210> 13
<211> 36
<212> DNA
<213> Artificial Sequence

<220>
<223> Description of Artificial Sequence:primer

<400> 13
ctggcggccg cttatgtcaa tgtgacttca actggg 36

<210> 14
<211> 23
<212> DNA
<213> Artificial Sequence

<220>
<223> Description of Artificial Sequence:primer

<400> 14
ctttaacgct gatgcctctt atg 23

<210> 15
<211> 25

<212> DNA
<213> Artificial Sequence

<220>
<223> Description of Artificial Sequence:primer

<400> 15
cataagaggc atcagcgtta aagtc 25

<210> 16
<211> 33
<212> DNA
<213> Artificial Sequence

<220>
<223> Description of Artificial Sequence:primer

<400> 16
cgacgacggt gccgaatctt gtggtaacga ttc 33

<210> 17
<211> 33
<212> DNA
<213> Artificial Sequence

<220>
<223> Description of Artificial Sequence:primer

<400> 17
gaatcgttac cacaagattc ggcaccgtcg tcg 33

<210> 18
<211> 33
<212> DNA
<213> Artificial Sequence

<220>
<223> Description of Artificial Sequence:primer

<400> 18
cgacgacggt tgcgaatctg ctggtaacga ttc 33

<210> 19
<211> 33
<212> DNA
<213> Artificial Sequence

<220>
<223> Description of Artificial Sequence:primer

<400> 19
gaatcgttac cagcagattc gcaaccgtcg tcg 33

<210> 20
<211> 29
<212> DNA
<213> Artificial Sequence

<220>
<223> Description of Artificial Sequence:primer

<400> 20
cctgctgttg ttgacttctc atcacactc 29

<210> 21
<211> 29
<212> DNA
<213> Artificial Sequence

<220>
<223> Description of Artificial Sequence:primer

<400> 21
gagtgtgatg agaagtcaac aacagcagg 29

<210> 22
<211> 37
<212> DNA
<213> Artificial Sequence

<220>
<223> Description of Artificial Sequence:primer

<400> 22
ctcaatggtc ttcaattcac ttgagaacat cttcaac 37

<210> 23
<211> 37
<212> DNA
<213> Artificial Sequence

<220>
<223> Description of Artificial Sequence:primer

<400> 23
gttgaagatg ttctcaagtg aattgaagac cattgag 37

<210> 24
<211> 37
<212> DNA
<213> Artificial Sequence

<220>
<223> Description of Artificial Sequence:primer

<400> 24
ctcaatggtc ttcaattgca ttgagaacat cttcaac 37

<210> 25
<211> 37
<212> DNA
<213> Artificial Sequence

<220>
<223> Description of Artificial Sequence:primer

<400> 25
gttgaagatg ttctcaatgc aattgaagac cattgag 37

<210> 26
<211> 36
<212> DNA
<213> Artificial Sequence

<220>
<223> Description of Artificial Sequence:primer

<400> 26
ggtcttcaat tagattgcac acatcttcaa ctttcc 36

<210> 27
<211> 36
<212> DNA
<213> Artificial Sequence

<220>
<223> Description of Artificial Sequence:primer

<400> 27
ggaaagttga agatgtgtgc aatctaattg aagacc 36

<210> 28
<211> 36
<212> DNA
<213> Artificial Sequence

<220>
<223> Description of Artificial Sequence:primer

<400> 28
ggtcttcaat tagattggca acatcttcaa ctttcc 36

<210> 29
<211> 36
<212> DNA
<213> Artificial Sequence

<220>
<223> Description of Artificial Sequence:primer

<400> 29
ggaaagttga agatgttgcc aatctaattg aagacc 36

<210> 30
<211> 39
<212> DNA
<213> Artificial Sequence

<220>
<223> Description of Artificial Sequence:primer

<400> 30
gtatctctcg agaaaagatt gaagtctgct gctttcaac 39

<210> 31
<211> 30
<212> DNA
<213> Artificial Sequence

<220>
<223> Description of Artificial Sequence:primer

<400> 31
caaagaaaga tacttaaact tgttcagacc 30

<210> 32
<211> 30
<212> DNA
<213> Artificial Sequence

<220>
<223> Description of Artificial Sequence:primer

<400> 32
ggtctgaaca agtttaagta tctttctttg 30

<210> 33
<211> 28
<212> DNA
<213> Artificial Sequence

<220>
<223> Description of Artificial Sequence:primer

<400> 33
ccaaggttaa agagaacgct atcgttgc 28

<210> 34
<211> 28
<212> DNA
<213> Artificial Sequence

<220>
<223> Description of Artificial Sequence:primer

<400> 34
gcaacgatag cgttctcttt aaccttgg 28

<210> 35
<211> 33
<212> DNA
<213> Artificial Sequence

<220>
<223> Description of Artificial Sequence:primer

<400> 35
cgacgacggt gccgaatctg ctggtaacga ttc 33

<210> 36
<211> 33
<212> DNA

<213> Artificial Sequence

<220>
<223> Description of Artificial Sequence:primer

<400> 36
gaatcgttac cagcagattc ggcaccgtcg tcg 33

<210> 37
<211> 42
<212> DNA
<213> Artificial Sequence

<220>
<223> Description of Artificial Sequence:primer

<400> 37
ctcaatggtc ttcaattgca ttgcacacat cttcaacttt cc 42

<210> 38
<211> 42
<212> DNA
<213> Artificial Sequence

<220>
<223> Description of Artificial Sequence:primer

<400> 38
ggaaagttga agatgtgtgc aatgcaattg aagaccattg ag 42

<210> 39
<211> 73
<212> DNA
<213> Artificial Sequence

<220>
<223> Description of Artificial Sequence:primer

<400> 39

cgaaaaatga gaggtactag attgatgggt ttgttattag ctttggctgg tttattacaa 60
ttaggtttgt ctc 73

<210> 40
<211> 75
<212> DNA
<213> Artificial Sequence

<220>
<223> Description of Artificial Sequence:primer

<400> 40

tcgagagaca aacctaattg taataaacca gccaaagcta ataacaaacc catcaatcta 60
gtacctctca ttttt 75

## Description

### Field of the invention

The present invention relates to the production of desoxyribonuclease with increased thermolability in non-animal host organisms. In particular, the present invention relates to variants of bovine pancreatic desoxyribonuclease I and their production. Also provided are use of bovine pancreatic desoxyribonuclease I variants and kits containing the same.

### Background of the invention

Bovine pancreatic desoxyribonuclease I is an industrial product with a wide range of applications. In the field of molecular biology and nucleic acid biochemistry, bovine pancreatic desoxyribonuclease I is used in applications such as nick translation, the production of random DNA fragments, desoxyribonuclease I protection assays such as transcription factor footprinting, removal of DNA template after in vitro transcription, removal of DNA from buffers and DNA polymerase enzyme preparations to be used in highly sensitive PCR applications, removal of DNA from RNA samples prior to applications such as RT-PCR, and removal of DNA from other preparations generated by biological and/or biochemical procedures, to name but a few (Sambrook, Fritsch & Maniatis, Molecular Cloning, A Laboratory Manual, 3rd edition, CSHL Press, 2001). Thus, degradation of DNA is effected by enzymatic hydrolysis of DNA catalysed by bovine pancreatic desoxyribonuclease I.

Bovine pancreatic desoxyribonuclease I has a molecular weight of about 30,000 daltons and an enzymatic activity optimum at pH 7.8. Bovine pancreatic desoxyribonuclease I hydrolyses phosphodiester linkages of DNA, preferentially adjacent to a pyrimidine nucleotide yielding DNA molecules with a free hydroxyl group at the 3' position and a phosphate group at the 5' position. The average chain length of a limit digest is a tetranucleotide. Moreover, like other desoxyribonucleases, bovine pancreatic desoxyribonuclease I is activated by divalent metal ions. Maximum activation is attained with $Mg^{2+}$ and $Ca^{2+}$. A metallosubstrate, such as a magnesium salt of DNA is necessary. Citrate completely inhibits magnesium-activated but not manganese-activated desoxyribonuclease I. Desoxyribonuclease I is inhibited by chelating agents such as EDTA, and by sodium dodecyl sulfate (Sambrook, Fritsch & Maniatis, Molecular Cloning, A Laboratory Manual, 3rd edition, CSHL Press, 2001).

In this document, the terms "variant of bovine pancreatic desoxyribonuclease I", "variant bovine pancreatic desoxyribonuclease I" and "bovine pancreatic desoxyribonuclease I variant" are used synonymously. They denote a protein that is a variant, i.e. an allelic form of the mature bovine pancreatic desoxyribonuclease I protein, generated by way of amino acid substitution.

For purposes of shorthand designation of bovine pancreatic desoxyribonuclease I variants described herein, it is noted that numbers refer to the amino acid residue/position along the amino acid sequence of putative mature bovine pancreatic desoxyribonuclease I as given in SEQ ID NO: 1. Amino acid identification uses the the three-letter abbreviations as well as the single-letter alphabet of amino acids, i.e., Asp D Aspartic acid, Ile I Isoleucine, Thr T Threonine, Leu L Leucine, Ser S Serine, Tyr Y Tyrosine, Glu E Glutamic acid, Phe F Phenylalanine, Pro P Proline, His H Histidine, Gly G Glycine, Lys K Lysine, Ala A Alanine, Arg R Arginine, Cys C Cysteine, Trp W Tryptophan, Val V Valine, Gln Q Glutamine, Met M Methionine, Asn N Asparagine. An amino acid at a particular position in an amino acid sequence is given by its three-letter abbreviation and a number. E.g., "Cys101" denotes the Cysteine residue at amino acid position 101 in SEQ ID NO: 1. A substitution of an amino acid residue by a different amino acid is given as the three-letter abbreviation added after the number indicating the position. E.g., "Cys101Ala" denotes the substitution of Cys at position 101 in SEQ ID NO: 1 by Ala.

The term "thermolabile" denotes an inactive or less active state, e.g. of a desoxyribonuclease enzyme and assayed like in Example 11, that is caused by a non-permissive temperature. Accordingly, compared to a first reference desoxyribonuclease, a second desoxyribonuclease with increased thermolability is characterised by a lower non-permissive temperature.

When desoxyribonuclease activity is quantified, the present document refers to "units" (U). Thus, the nucleolytic activity of bovine pancreatic desoxyribonuclease I and variants thereof is quantified using a photometric assay similar to the assay published by Kunitz (Kunitz, M., J. Gen. Physiol. 33 (1950) 349-62 and 363). The "specific desoxyribonuclease activity" or "specific activity" of a given preparation is defined as the number of units per mg of protein in the preparation, determined by the method described in detail in Example 11.

A "methylotrophic yeast" is defined as a yeast that is capable of utilising methanol as its carbon source. The term also comprises laboratory strains thereof. In case a methylotrophic yeast strain is auxotrophic and because of this needs to be supplemented with an auxillary carbon-containing substance such as, e.g. histidine in the case of a methylotrophic yeast strain unable to synthesise this amino acid in sufficient amounts, this auxillary substance is regarded as a nutrient but not as a carbon source.

A "vector" is defined as a DNA which can comprise, i.e. carry, and maintain the DNA fragment of the invention, including, for example, phages and plasmids. These terms are understood by those of skill in the art of genetic engineering. The term "expression cassette" denotes a nucleotide sequence encoding a pre-protein, operably linked to a promoter and a terminator. As for vectors containing an expression cassette, the terms "vector" and "expression vector" are used as synonyms.

The term "oligonucleotide" is used for a nucleic acid molecule, DNA (or RNA), with less than 100 nucleotides in length.

"Transformation" means introducing DNA into an organism, i.e. a host organism, so that the DNA is replicable, either as an extrachromosomal element or by chromosomal integration.

The term "expression" and the verb "to express" denote transcription of DNA sequences and/or the translation of the transcribed mRNA in a host organism resulting in a pre-protein, i.e. not including post-translational processes.

A nucleotide sequence "encodes" a peptide or protein when at least a portion of the nucleic acid, or its complement, can be directly translated to provide the amino acid sequence of the peptide or protein, or when the isolated nucleic acid can be used, alone or as part of an expression vector, to express the peptide or protein in vitro, in a prokaryotic host cell, or in a eukaryotic host cell.

A "promoter" is a regulatory nucleotide sequence that stimulates transcription. These terms are understood by those of skill in the art of genetic engineering. Like a promoter, a "promoter element" stimulates transcription but constitutes a sub-fragment of a larger promoter sequence.

The term "operably linked" refers to the association of two or more nucleic acid fragments on a single vector so that the function of one is affected by the other. For example, a promoter is operably linked with a coding sequence, i.e. a nucleotide sequence encoding a protein or a pre-protein, when it is capable of affecting the expression of that coding sequence, i.e., that the coding sequence is under the transcriptional control of the promoter.

The term "polypeptide" or "protein" denotes a polymer composed of more than 90 amino acid monomers joined by peptide bonds. The term "peptide" denotes an oligomer composed of 90 or fewer amino acid monomers joined by peptide bonds. A "peptide bond" is a covalent bond between two amino acids in which the $\alpha$-amino group of one amino acid is bonded to the $\alpha$-carboxyl group of the other amino acid.

The term "pre-protein" or "pre-protein form" denotes a primary translation product that is a precursor of a mature protein, i.e. in this case a protein results from post-translational processing of a pre-protein.

The term "post-translational processing" denotes the modification steps a pre-protein is subjected to, in order result in a mature protein in a cellular or extracellular compartment.

A "signal peptide" is a cleavable signal sequence of amino acids present in the pre-protein form of a secretable protein. Proteins transported across the cell membrane, i.e. "secreted", typically have an N- terminal sequence rich in hydrophobic amino acids, typically about 15 to 30 amino acids long. Sometime during the process of passing through the membrane, the signal sequence is cleaved by a signal peptidase (Alberts, B., Johnson, A., Lewis, J., Raff, M., Roberts, K., Walter, P. (eds), Molecular Biology of the Cell, fourth edition, 2002, Garland Science Publishing). Many sources of signal peptides are well known to those skilled in the art and can include, for example, the amino acid sequence of the $\alpha$-factor signal peptide from Saccharomyces cerevisiae and the like. Another example is the bovine signal peptide of the native bovine pancreatic desoxyribonuclease I pre-protein. In general, the pre-protein N-terminus of essentially any secreted protein is a potential source of a signal peptide suitable for use in the present invention. A signal peptide can also be bipartite comprising two signal peptides directing the pre-protein to a first and a second cellular compartment. Bipartite signal peptides are cleaved off stepwise during the course of the secretory pathway. A specific example therefor is the prepro peptide of the $\alpha$-factor from Saccharomyces cerevisiae (Waters et al., J. Biol. Chem. 263 (1988) 6209-14).

Pre-proteins with an N-terminal signal peptide are directed to enter the "secretory pathway". The secretory pathway comprises the processes of post-translational processing and finally results in secretion of a protein. Glycosylation and the formation of disulfide bonds are processes that are part of the secretory pathway prior to secretion. In the present document it is understood that proteins secreted by methylotrophic yeast strains have passed through the secretory pathway.

The preferred way to inactivate bovine pancreatic desoxyribonuclease I, i.e reduce desoxyribonuclease activity to approximately zero units per mg of bovine pancreatic desoxyribonuclease I protein, is heat treatment (Alberts, B., Johnson, A., Lewis, J., Raff, M., Roberts, K., Walter, P. (eds), Molecular Biology of the Cell, fourth edition, 2002, Garland Science Publishing). However, conventional bovine pancreatic desoxyribonuclease I prepared from pancreatic tissue is substantially heat-stable. After heat incubation at 95°C for, e.g., 30 min there is residual desoxyribonuclease activity that may even increase again once the incubation temperature is decreased (Hanaki K. et al. Biotechniques 29 (2000) 38-42). Irreversible heat inactivation can be accomplished at 95°C when the concentration of $MgCl_2$ in the bovine pancreatic desoxyribonuclease I-containing sample is increased to 6 mM (Bickler et al., Biotechniques 13 (1992) 64-66). Heat treatment is potentially deleterious for other substances that may be comprised in the bovine pancreatic desoxyribonuclease I-containing sample, such as RNA or protein. Therefore, a lower inactivation temperature is desired.

To the knowledge of the inventors, no bovine desoxyribonucleases with increased thermolability are known so far. Attempts were made to identify desoxyribonucleases with increased thermolability in other organisms. A potential alternative source for desoxyribonuclease is shrimp. A shrimp desoxyribonuclease I was purified from Penaeus japonicus and was characterised in more detail (Wang W.-Y. et al. Biochem J. 346 (2000) 799-804). It was found that the shrimp desoxyribonuclease I was, however distantly, evolutionary related to nucleases, i.e. enzymes capable of hydrolysing both DNA and RNA. Accordingly, experiments showed that shrimp desoxyribonuclease I from Penaeus japonicus also has ribonuclease activity. In contrast, no ribonuclease activity was detected in bovine pancreatic desoxyribonuclease I.

US patent application 2002/0042052 A1 describes a desoxyribonuclease from another shrimp species, that is Pandalus borealis. The desoxyribonuclease from Pandalus borealis is characterised by an increased thermolability. It can be purified e.g. from shrimp processing water, a by-product of the shrimp fishing industry. According to the document, purified shrimp desoxyribonuclease can be inactivated by incubation for 2 min at 94°C. Although the document mentions RT-PCR, i.e. the polymerase chain reaction that uses RNA as a first template, the document is completely silent about any potential ribonuclease activity of the Pandalus borealis desoxyribonuclease I. No example for application of the desoxyribonuclease from Pandalus borealis in RT-PCR experiments is shown. Additionally, no data are provided regarding the actual amino acid sequence of the Pandalus borealis desoxyribonuclease I as well as the species homogeneity of the source from which the desoxyribonuclease with increased thermolability was purified.

The desoxyribonucleases, particularly the desoxyribonucleases with increased thermolability known to the art have certain disadvantages. The present invention provides improved desoxyribonucleases with increased thermolability. The improved desoxyribonucleases of the invention are variants, by means of amino acid substitution, of bovine pancreatic desoxyribonuclease I. Furthermore, said variants lack any detectable ribonuclease activity.

It is therefore an object of the invention to provide a desoxyribonuclease with increased thermolability compared to bovine pancreatic desoxyribonuclease I. It is a further object of the invention to provide a desoxyribonuclease that can be inactivated at temperatures below 95°C. It is a further object of the invention to provide a desoxyribonuclease without any detectable ribonuclease activity. It is a further object of the invention to provide a cost-effective method to produce the desoxyribonuclease as a recombinant protein synthesised by a non-animal host organism. Another object of the invention is to provide an expression system which simplifies and accelerates the separation of the desoxyribonuclease from cellular or media components. Yet another object of the invention is that the production procedure is amenable to upscaling towards a cost-effective industrial process.

## Brief summary of the invention

It was surprisingly found that thermolability of bovine pancreatic desoxyribonuclease I can be increased specifically by way of amino acid substitution at certain positions in the amino acid sequence of bovine pancreatic desoxyribonuclease I. Specific increase of thermolability means that at the same time, enzymatic activity is preserved, that is desoxyribonuclease activity of such variants of bovine pancreatic desoxyribonuclease I remains intact, however at decreased levels.

According to the invention, the desoxyribonuclease with increased thermolability is a variant, by way of amino acid substitution, of bovine pancreatic desoxyribonuclease I, wherein at least one different amino acid substitutes for an amino acid residue, that is at least one of the amino acid residues of bovine pancreatic desoxyribonuclease I selected from the group consisting of Cys173, Cys101, Cys104, Lys117, Arg185, Arg187, Ile3, Phe82, and Phe128, numbered from the N-terminus of the 260-amino acid bovine pancreatic desoxyribonuclease I according to SEQ ID NO: 2 (wild-type reference), to form a bovine pancreatic desoxyribonuclease I variant with desoxyribonuclease activity and increased thermolability compared to the wild-type reference whereby the different amino acid is selected from the group consisting of

- Ala, Ser, Thr, Gly, and Val when the different amino acid substitutes for a Cys 173, Cys101 or Cys104 residue,
- Asp, Glu, Asn, Gln, and Ile when the different amino acid substitutes for the Lys 117 residue,
- His, Ala, Asn, and Gln when the different amino acid substitutes for an Arg185 or Arg187 residue,
- Ala, Ser, Thr, Gly, and Val when the different amino acid substitutes for the Ile3 residue,
- Asn, Gln, and Ile when the different amino acid substitutes for a Phe82 or Phe128 residue.

Moreover, according to the invention, the specific desoxyribonuclease activity of the variant of bovine pancreatic desoxyribonuclease I is approximately zero units per mg of protein following heating of the variant of bovine pancreatic desoxyribonuclease I for about 5 min at a temperature below 95°C, that is between 94°C and 70°C. In addition, according to the invention, the variant of bovine pancreatic desoxyribonuclease I has no measurable ribonuclease activity.

## Detailed description of the invention

Based on the crystal structure of bovine pancreatic desoxyribonuclease I at a resolution of 2Å (Suck, D. et al.,

Nature 332 (1988) 464-468; Lahm, A. & Suck, D., J. Mol. Biol. 221 (1991) 645-667) positions in the amino acid sequence of the mature, i.e. secreted bovine pancreatic desoxyribonuclease I were identified as potential targets for amino acid substitution. Amino acid substitution was preferably considered at positions where amino acids participating in intramolecular interactions were located. The amino acids relevant in this respect generally were (a) a first amino acid residue interacting with a second amino acid residue by electrostatic forces, (b) a first amino acid residue interacting with a second amino acid residue by van-der-Waals forces, (c) a first amino acid residue and a second amino acid residue interacting with the same divalent metal ion (d) a first cysteine residue and a second cysteine residue joined by a disulfide bond. Furthermore, those amino acids that were located distant from the domains interacting with DNA were considered for substitution with even higher preference.

A person skilled in the art is well aware of methods to substitute one ore more amino acid residues in a protein. For the present invention, synthetic nucleotide sequences encoding variants of bovine pancreatic desoxyribonuclease I were synthesised and expressed in microbial host organisms. The preferred method, however, was to synthesise variants of bovine pancreatic desoxyribonuclease I that were expressed and secreted by methylotrophic yeast strains. Using this strategy, subsequent steps to characterise the variants of bovine pancreatic desoxyribonuclease I could be performed more efficiently (see Examples 1 to 6).

The variants of bovine pancreatic desoxyribonuclease I that were generated were compared to the reference, i.e. wild-type bovine pancreatic desoxyribonuclease I that served as a starting point for the amino acid substitutions. Two parameters were compared, thermolability and specific desoxyribonuclease activity. Variants of bovine pancreatic desoxyribonuclease I were desired that showed a combination of increased thermolability and sufficient residual desoxyribonuclease activity. Regarding thermolability it was desired that heat incubation for 5 min at a temperature below 95°C, that is between 94°C and 70°C, inactivated the variants of bovine pancreatic desoxyribonuclease I. Regarding desoxyribonuclease enzymatic activity, lower than wild-type levels of specific activity, that is desoxyribonuclease activity per mg of protein, were regarded to be acceptable if the specific activity was still above 50% of the wild-type level.

It was found that in the amino acid sequence of bovine pancreatic desoxyribonuclease I the amino acid residues Cys173, Cys101, Cys104, Lys117, Arg185, Arg187, Ile3, Phe82, and Phe128 could be substituted by a different amino acid, in order to obtain a variant of bovine pancreatic desoxyribonuclease I with the desired properties. Particular amino acid substitutions were combined in double mutant or triple-mutant variants of bovine pancreatic desoxyribonuclease I, thereby further increasing thermolability.

Variants of bovine pancreatic desoxyribonuclease I were preferably produced as heterologous proteins in microbial host organisms such as bacteria and fungi. The person skilled in the art is well aware of bacterial expression systems that exist for a variety of prokaryotic hosts such as E. coli, Bacillus and Staphylococcus species, to name but a few. Even more preferred microbial host organisms are fungi. An example for a preferred fungal genus is Aspergillus. Yet, even more preferred are yeast species such as species of the genera Saccharomyces or Schizosaccharomyces. Yet, even more preferred are strains of methylotrophic yeast species.

Methylotrophic yeasts have the biochemical pathways necessary for methanol utilization and are classified into four genera, based upon cell morphology and growth characteristics: Hansenula, Pichia, Candida, and Torulopsis. The most highly developed methylotrophic host systems utilize Pichia pastoris (Komagataella pastoris) and Hansenula polymorpha (Pichia angusta).

Expression of heterologous proteins in yeast is described in US 5,618,676, US 5,854,018, US 5,856,123, and US 5,919,651.

Yeast organisms produce a number of proteins that are synthesized intracellularly but have a function outside the cell. These extracellular proteins are referred to as secreted proteins. Initially the secreted proteins are expressed inside the cell in the form of a precursor or a pre-protein containing an N-terminal signal peptide ensuring effective direction of the expressed product into the secretory pathway of the cell, across the membrane of the endoplasmic reticulum. The signal peptide is generally cleaved off from the desired product during translocation. Cleavage is effected proteolytically by a signal peptidase. A particular sub-sequence of amino acids of the signal peptide is recognised and cleaved by the signal peptidase. This sub-sequence is referred to as signal peptidase cleavage site. Once having entered the secretory pathway, the protein is transported to the Golgi apparatus. From the Golgi apparatus the proteins are distributed to the plasma membrane, lysosomes and secretory vesicles.

Secreted proteins are confronted with different environmental conditions as opposed to intracellular proteins. Part of the processes of the secretory pathway is to stabilise the maturing extracellular proteins. Therefore, pre-proteins that are passed through the secretory pathway of yeast undergo specific posttranslational processing. For example, processing can comprise the generation of disulfide bonds to form intramolecular crosslinks. Moreover, certain amino acids of the protein can be glycosylated.

Several approaches have been suggested for the expression and secretion in yeast of proteins heterologous to yeast. EP 0 116 201 describes a process by which proteins heterologous to yeast are transformed by an expression vector harboring DNA encoding the desired protein, a signal peptide and a peptide acting as a signal peptidase cleavage site. A culture of the transformed organism is prepared and grown, and the protein is recovered from culture media. For use in yeast cells a suitable signal peptide has been found to be the α-factor signal peptide from Saccharomyces cerevisiae (US 4,870,008).

When the present invention was made it was found surprisingly that the bovine signal peptide of the native bovine pancreatic desoxyribonuclease I pre-protein is also sufficient to direct the pre-protein to the secretory pathway of methylotrophic yeast. Therefore, the bovine signal peptide of the native bovine pancreatic desoxyribonuclease I pre-protein can be used to express and secrete a heterologous gene product in methylotrophic yeast.

During secretion, the yeast enzyme KEX-2 is the signal peptidase which recognizes a Lysine-Arginine sequence as its cleavage site in the pre-protein. KEX-2 cleaves at the junction to the sequence of the desired protein. As a result, the desired gene product is released and free of the leader portions, i.e. the signal peptide of the pre-protein. KEX-2 endoprotease was originally characterised in Saccharomyces yeast where it specifically processes the precursor of mating type α-factor and a killer factor (Julius, D., et al., Cell 37 (1984) 1075-1089). Methylotrophic yeast species such as Pichia pastoris share the KEX-2-type protease (similar role and function) with Saccharomyces cerevisiae (Werten, M.W., et al., Yeast 15 (1999) 1087-1096).

A well-established methylotrophic yeast species exemplarily described as host for high-level recombinant protein expression is Pichia pastoris (US 4,683,293, US 4,808,537, US 4,812,405, US 4,818,700, US 4,837,148, US 4,855,231, US 4,857,467, US 4,879,231, US 4,882,279, US 4,885,242, US 4,895,800, US 4,929,555, US 5,002,876, US 5,004,688, US 5,032,516, US 5,122,465, US 5,135,868, US 5,166,329, WO 00/56903). In the absence of glucose, Pichia pastoris uses methanol as a carbon source which at the same time is a hallmark of a methylotrophic organism. The alcohol oxidase (AOX1) promoter given in SEQ ID NO: 11 controls expression of alcohol oxidase, which catalyses the first step in methanol metabolism. Typically, 30% of the total soluble protein in methanol-induced cells is alcohol oxidase. Several Pichia expression vectors carry the AOX1 promoter and use methanol to induce high-level expression of desired heterologous proteins. Expression constructs also integrate into the Pichia pastoris genome, creating a transformed and genetically stable host.

Using an expression vector encoding a heterologous pre-protein comprising a signal peptide or a signal peptide with a signal peptidase cleavage site, and a desired protein, methylotrophic yeast strains such as Pichia pastoris strains can be manipulated in order to secrete the desired product into the growth medium from which the secreted protein can be purified. It may be advantageous to produce nucleotide sequences encoding the pre-protein possessing a substantially different codon usage. Codons may be selected to increase the rate at which expression of the pre-protein occurs in a particular yeast expression host in accordance with the frequency with which particular codons are utilised by the host. Other reasons for substantially altering the nucleotide sequence encoding the pre-protein, without altering the encoded amino acid sequences, include the production of RNA transcripts having more desirable properties, such as a greater half-life, than transcripts produced from the naturally occurring sequence.

Using a vector comprising the nucleotide sequence encoding the pre-protein that is competent for expression, e.g. operably linked to a promoter or promoter element and to a terminator or terminator element, as well as to sequences required for efficient translation, the host organism is transformed with a vector, and transformants are selected. Transformants are then analysed with respect to the yield of recombinant protein secreted into the growth medium. Transformants secreting the highest quantities of enzymatically active recombinant protein are selected. Thus, transformants secreting variants of bovine pancreatic desoxyribonuclease I with desoxyribonuclease activity are selected.

On the one hand, expression yield is dependent on proper targeting of the desired product, e.g. to the secretory pathway by means of a signal peptide such as the α-factor signal peptide from Saccharomyces cerevisiae or the bovine signal peptide of the native bovine pancreatic desoxyribonuclease I pre-protein. On the other hand, expression yield can be increased by increasing the dosage of the gene encoding the desired product. Thus, the copy number of the expression construct, that is the expression vector or the expression cassette, in the host organism is amplified. One way to accomplish this is by multiple transformation of an expression vector encoding the desired product. Another way is to introduce the gene encoding the desired product into the host organism using a first and a second expression vector, whereby the second expression vector is based on a selectable marker which differs from the selectable marker used in the first expression vector. The second expression vector encoding the same desired product can even be introduced when the host organism already carries multiple copies of a first expression vector (US 5,324,639; Thill, G.P., et al., Positive and Negative Effects of Multi-Copy Integrated Expression in Pichia pastoris, International Symposium on the Genentics of Microorganisms 2 (1990), pp. 477-490; Vedvick, T., et al., J. Ind. Microbiol. 7 (1991) 197-201; Werten, M.W., et al., Yeast 15 (1999) 1087-1096).

Secretion of an expressed variant of bovine pancreatic desoxyribonuclease I into the growth medium directs the mature recombinant protein to the extracytoplasmic space from where it diffuses into growth media. Thus, transformed methylotrophic yeast grown in liquid culture secretes the bovine pancreatic desoxyribonuclease I variant into the liquid growth medium, i.e. the liquid culture medium. This allows a very efficient separation of yeast biomass from the recombinant protein using, e.g. filtration techniques. As a result, a bovine pancreatic desoxyribonuclease I variant purified from this source is very efficiently separated from other enzyme activities such as ribonuclease or protease activities.

Therefore, a first preferred embodiment of the invention is a variant, by way of amino acid substitution, of bovine pancreatic desoxyribonuclease I, wherein at least one different amino acid substitutes for an amino acid residue, that is at least one of the amino acid residues of bovine pancreatic desoxyribonuclease I selected from the group consisting of Cys173, Cys101, Cys104, Lys117, Arg185, Arg187, Ile3, Phe82, and Phe128, numbered from the N-terminus of the 260-amino acid bovine pancreatic desoxyribonuclease I according to SEQ ID NO: 2 (wild-type reference), to form a bovine pancreatic desoxyribonuclease I variant with desoxyribonuclease activity and increased thermolability compared to the wild-type reference whereby the different amino acid is selected from the group consisting of

- Ala, Ser, Thr, Gly, and Val when the different amino acid substitutes for a Cys173, Cys101 or Cys104 residue,
- Asp Glu, Asn, Gln, and Ile when the different amino acid substitutes for the Lys117 residue,
- His Ala, Asn, and Gln when the different amino acid substitutes for an Arg185 or Arg187 residue,
- Ala Ser, Thr, Gly, and Val when the different amino acid substitutes for the Ile3 residue,
- Asn Gln, and Ile when the different amino acid substitutes for a Phe82 or Phe128 residue.

In another preferred embodiment of the invention, two different amino acids substitute for two amino acid residues, whereby the first different amino acid is Ala that substitutes for Cys101, and the second different amino acid is Ala that substitutes for Cys104. In yet another preferred embodiment of the invention, two different amino acids substitute for two amino acid residues, whereby the first different amino acid is Ala that substitutes for Arg185, and the second different amino acid is His that substitutes for Arg187. In yet another preferred embodiment of the invention, three different amino acids substitute for three amino acid residues, whereby the first different amino acid is Asp that substitutes for Lys117, the second different amino acid is Ala that substitutes for Arg185, and the third different amino acid is His that substitutes for Arg187.

In a further preferred embodiment of the invention, the specific desoxyribonuclease activity of the variant of bovine pancreatic desoxyribonuclease I is approximately zero units per mg of protein following heating of the variant of bovine pancreatic desoxyribonuclease I for about 5 min at a temperature less than 95°C. In another preferred embodiment of the invention, the specific desoxyribonuclease activity of the variant of bovine pancreatic desoxyribonuclease I is approximately zero units per mg of protein following heating of the variant of bovine pancreatic desoxyribonuclease I for about 5 min at a temperature between 94°C and 71°C. In another preferred embodiment of the invention, the specific desoxyribonuclease activity of the variant of bovine pancreatic desoxyribonuclease I is approximately zero units per mg of protein following heating of the variant of bovine pancreatic desoxyribonuclease I for about 5 min at a temperature of approximately 70°C. In yet another preferred embodiment of the invention, the specific desoxyribonuclease activity of the variant of bovine pancreatic desoxyribonuclease I is 100% or less than that of bovine pancreatic desoxyribonuclease I. Thus, when produced and purified under equivalent conditions, the specific desoxyribonuclease activity of the variant of bovine pancreatic desoxyribonuclease I is 100% or less when compared to the unchanged bovine pancreatic desoxyribonuclease I, that is the wild-type form. In yet another preferred embodiment of the invention, the specific desoxyribonuclease activity of the variant of bovine pancreatic desoxyribonuclease I is at least 50% compared to that of bovine pancreatic desoxyribonuclease I. Thus, when produced and purified under equivalent conditions, the specific desoxyribonuclease activity of the variant of bovine pancreatic desoxyribonuclease I is decreased when compared to the unchanged bovine pancreatic desoxyribonuclease I, that is the wild-type form.

Another preferred embodiment of the invention is a method to produce a variant of bovine pancreatic desoxyribonuclease I comprising the steps of (a) providing a vector comprising a nucleotide sequence that encodes the variant of bovine pancreatic desoxyribonuclease I, (b) transforming a microbial host strain with the vector, (c) cultivating the transformed microbial host strain in a growth medium that contains nutrients, whereby the microbial host strain expresses the variant of bovine pancreatic desoxyribonuclease I, and (d) purifying the variant of bovine pancreatic desoxyribonuclease I from the microbial host strain and/or the growth medium.

Translation efficiency of a heterologous protein can be improved by adapting the codons of the nucleotide sequence encoding the heterologous protein according to the preferred codons in the host organism. Thus, in a very preferred embodiment of the invention, the nucleotide sequence that encodes the variant of bovine pancreatic desoxyribonuclease I is SEQ ID NO: 3.

In an even more preferred embodiment of the invention, (a) the vector comprises a nucleotide sequence that encodes a pre-protein consisting of the bovine pancreatic desoxyribonuclease I and a signal peptide, (b) the microbial host strain is a methylotrophic yeast strain, (c) the growth medium contains methanol as a carbon source, (d) the methylotrophic yeast strain expresses and secretes the variant of bovine pancreatic desoxyribonuclease I, and (e) the variant of bovine pancreatic desoxyribonuclease I is purified from the growth medium. In yet another very preferred embodiment of the invention, the nucleotide sequence is SEQ ID NO: 3. In yet another very preferred embodiment of the invention, the signal peptide contains a signal peptidase cleavage site which is located directly adjacent to the first amino acid of the variant of bovine pancreatic desoxyribonuclease 1. In yet another very preferred embodiment of the invention, the amino acid sequence of the expressed pre-protein is selected from the group consisting of (a) SEQ ID NO: 8, (b) SEQ ID NO: 9, and (c) SEQ ID NO: 10. In yet another very preferred embodiment of the invention, the nucleotide sequence encoding the variant of bovine pancreatic desoxyribonuclease I is SEQ ID NO: 6. In yet another very preferred embodiment of the invention, the nucleotide sequence encoding the pre-protein consists of the nucleotide sequence encoding the signal peptide fused to the nucleotide sequence encoding the variant of bovine pancreatic desoxyribonuclease I. In yet another very preferred embodiment of the invention, the nucleotide sequence encoding the signal peptide is selected from the group consisting of (a) SEQ ID NO: 5, (b) SEQ ID NO: 6, and (c) SEQ ID NO: 7. SEQ ID NO: 5 is the nucleotide sequence encoding the amino acid sequence of the signal peptide of the native bovine pancreatic DNase I pre-protein. SEQ ID NO: 6 is the nucleotide sequence encoding the amino acid sequence of the signal peptide of the native bovine pancreatic DNase I pre-protein and an additional signal peptidase cleavage site. SEQ ID NO: 7 is the nucleotide sequence encoding the amino acid sequence of the signal peptide of the α-factor from Saccharomyces cerevisiae. This signal peptide is a bipartite signal peptide.

Yeast-derived as well as non-yeast-derived eukaryotic signal peptides other than those particularly mentioned can be used for the same purpose. Although the signal peptides might not be cleavable by the signal peptidase, a signal peptidase cleavage peptide can be inserted into the pre-protein amino acid sequence, that is between the amino acid sequence of the signal peptide and the amino acid sequence of the variant bovine pancreatic desoxyribonuclease I polypeptide. Therefore, in yet another very preferred embodiment of the invention, the signal peptide contains a signal peptidase cleavage site which is located directly adjacent to the first amino acid of the bovine pancreatic protein.

In another preferred embodiment of the invention, the nucleotide sequence encoding the pre-protein is operably linked to a promoter or promoter element. It is preferred that the vector is a plasmid capable of being replicated as an episome in the methylotrophic yeast strain. It is furthermore preferred that an artificial chromosome capable of being replicated in the methylotrophic yeast strain contains the vector. Yet, it is very much preferred that a chromosome of the methylotrophic yeast strain contains the vector.

Thus, in the preferred method using methylotrophic yeast strains and particularly in Pichia pastoris strains, the vector encodes an amino acid sequences for a variant of bovine pancreatic desoxyribonuclease I pre-protein that enters the secretory pathway.

In a further preferred embodiment of the invention, the methylotrophic yeast strain is a Hansenula, Pichia, Candida or Torulopsis species. It is very preferred that the methylotrophic yeast strain is selected from the group consisting of Pichia pastoris, Hansenula polymorpha, Candida boidinii and Torulopsis glabrata. It is even more preferred that the methylotrophic yeast strain is the Pichia pastoris strain with the American Type Culture Collection accesssion number 76273 or a derivative thereof.

Another preferred embodiment of the invention is a Pichia pastoris strain with a chromosome that contains a vector comprising a nucleotide sequence that encodes a pre-protein consisting of the variant of bovine pancreatic desoxyribonuclease I and a signal peptide, operably linked with the Pichia pastoris AOX1 promoter according to SEQ ID NO: 11 or a promoter element thereof, whereby the nucleotide sequence that encodes the pre-protein is SEQ ID NO: 6 or SEQ ID NO: 7, fused to SEQ ID NO: 3.

The person skilled in the art is aware of the fact that the yield of secreted heterologous protein, such as a variant of bovine pancreatic desoxyribonuclease I, obtainable from growth medium, such as liquid growth medium, can be increased when the number of copies of the nucleotide sequence encoding the pre-protein from which the heterologous protein is expressed and secreted, is increased. Thus, the yield of secreted heterologous protein obtainable from growth medium can be increased when number of copies of the vector in the genome of the methylotrophic yeast strain is increased. For example, the copy number of the vector can be increased by subjecting the methylotrophic yeast strain to repeated transformations of the vector and repeated selection rounds using increasing concentrations of the selective agent against which the selective marker comprised in the vector confers resistance (US 5,324,639; Thill, G.P., et al., Positive and Negative Effects of Multi-Copy Integrated Expression in Pichia pastoris, International Symposium on the Genentics of Microorganisms 2 (1990), pp. 477-490; Vedvick, T., et al., J. Ind. Microbiol. 7 (1991) 197-201).

The person skilled in the art is also aware of the fact that repeated transformations can be carried out using more than one vector. For example, repeated transformations can be carried out using a first and a second vector, whereby the first and the second vector encode the same pre-protein, whereby in the first and in the second vector the nucleotide sequence encoding the pre-protein is operably linked to a promoter or promoter element, whereby the same variant bovine pancreatic desoxyribonuclease I is expressed and secreted, and whereby the first and the second vector confer resistance to a first and a second selection marker.

An example for a first selective marker is the Sh ble gene, that is the Zeocin™ resistance gene (Drocourt, D., et al., Nucleic Acids Res. 18 (1990) 4009; Carmels, T., et al., Curr. Genet. 20 (1991) 309-314). The protein encoded by the Sh ble gene binds Zeocin™ stoichiometrically and with a strong affinity. The binding of Zeocin™ inhibits its toxic activity thereby selecting for transformants containing the Sh ble gene. It is known to a person skilled in the art that increasing the concentration of Zeocin™ as the selective agent in the medium selects for an increase in the number of copies of the vector expressing the Sh ble gene. It is therefore advantageous to use a vector with the Sh ble gene as a selectable marker to generate by repeated transformation multiple transformants of the methylotrophic yeast strain containing multiple copies of the vector. It is furthermore advantageous that transformations are repeated and selection for even more resistant transformants is repeated until for the transformed methylotrophic yeast strain no further increase of the level of resistance to Zeocin™ is obtained anymore or no further increase of the Zeocin™ concentration in the selection medium is possible anymore.

In case a first and a second vector are used, an example for a second selection marker is resistance against aminoglycoside antibiotics (Southern, P.J., and Berg, P., J. Mol. Appl. Genet. 1 (1982) 327-341) such as G418. Thus, an exemplary second vector expresses a resistance gene that confers resistance against G418. For example, there are several aminoglycoside phosphotransferases known to the art that confer resistance to aminoglycoside antibiotics (van Treeck, U., et al., Antimicrob Agents Chemother. 19 (1981) 371-380; Beck, E., et al., Gene 19 (1982) 327-336). The aminoglycoside phosphotransferase I (APH-I) enzyme has the ability to inactivate the antibiotic G418 and is an established selectable marker in yeast (Chen, X.J., and Fukuhara, H., Gene (1988) 181-192).

Thus, for the purpose of further increasing the dosage of the nucleotide sequence encoding the pre-protein from which the variant of bovine pancreatic desoxyribonuclease I is expressed and secreted, the second vector is advantageously used for further rounds of transformation and selection, whereby in this case a preferred selective agent is G418 and whereby for transformation of the methylotrophic yeast strain the first vector is used.

A person skilled in the art is familiar with the purification of bovine pancreatic desoxyribonuclease I by means of chromatography (Funakoshi, A., et al., J. Biochem. (Tokyo) 88 (1980) 1113-1138; Paudel, H.K., and Liao, T.H., J. Biol Chem. 261 (1986) 16006-16011; Nefsky, B., and Bretscher, A., Eur. J. Biochem. 179 (1989) 215-219). Principally, the purification of a variant of a bovine pancreatic desoxyribonuclease I can be accomplished accordingly. It is preferred, however, that a variant of bovine pancreatic desoxyribonuclease I which has been secreted by a transformed methylotrophic yeast strain into the growth medium is purified using ion exchange chromatography. Also very preferred is a further purification step consisting of affinity chromatography using heparin sepharose. Using this further step, a person skilled in the art is able to achieve about 98% purity of variant bovine pancreatic desoxyribonuclease I, to be tested by means of SDS PAGE, whereby gels are stained using Coomassie Blue.

Yet, another preferred embodiment of the invention is a variant of bovine pancreatic desoxyribonuclease I, by one of the methods described above. A further preferred embodiment of the invention is the use of a variant of bovine pancreatic desoxyribonuclease I for hydrolysing DNA and subsequently reducing the specific desoxyribonuclease activity of the variant of bovine pancreatic desoxyribonuclease I to approximately zero units per mg of protein by heating of the variant of bovine pancreatic desoxyribonuclease I for about 5 min at a temperature less than 95°C. Also very much preferred is the use of a variant of bovine pancreatic desoxyribonuclease I, characterised in that the specific desoxyribonuclease activity of the variant of bovine pancreatic desoxyribonuclease I is reduced to approximately zero units per mg of protein by heating of the variant of bovine pancreatic desoxyribonuclease I for about 5 min at a temperature of approximately 70°C.

Yet, another preferred embodiment of the invention is a kit of parts containing the variant of bovine pancreatic desoacyribonuclease I and a reaction buffer comprising a divalent cation. It is also preferred that the variant of bovine pancreatic desoxyribonuclease I is dissolved in a buffer containing 2 mM TrisHCl, 2 mM $MgCl_2$, 4 mM $CaCl_2$, 50% glycerol, pH 7.6, and the ten times concentrated reaction buffer contains 100 mM TrisHCl pH 7.5, 100 mM $MgCl_2$, and 10 mM dithioerythritol.

The following examples, references, sequence listing and figures are provided to aid the understanding of the present invention, the true scope of which is set forth in the appended claims. It is understood that modifications can be made in the procedures set forth without departing from the spirit of the invention.

## Description of the Figures

Figure 1 Exemplary map of the plasmid pDNM34-1 which is a derivative of the commercially available plasmid pPICZαA (Invitrogen) that confers resistance to Zeocin™. The insert denoted "DNAseC173A" is the synthetic DNA sequence encoding the variant of bovine secreted desoxyribonuclease I that carries the Cys173Ala amino acid substitution, and that is fused to the nucleotide sequence encoding the α-factor signal peptide from Saccharomyces cerevisiae. "AOX1-Prom" denotes the Pichia pastoris AOX1 promoter, "Term" denotes the Pichia pastoris AOX1 terminator. Other pDNM#-1 derivatives described in Example 3 differed with respect to the amino acid substitution encoded in the synthetic DNA sequence encoding the respective variant of bovine secreted desoxyribonuclease I. The corresponding pDNM#-3 vectors derived from pPICZA (Invitrogen) lack the α-factor signal peptide from Saccharomyces cerevisiae (Sfu I - Xho I fragment) but instead have the bovine signal peptide of the native bovine pancreatic desoxyribonuclease I pre-protein described in Example 1 inserted at the same position.

Figure 2 Map of the plasmid pDNM34-2 which is a derivative of the commercially available plasmid pPIC9K (Invitrogen) that confers resistance to G418. The insert denoted "DNAseC173A" is the synthetic DNA sequence encoding the variant of bovine secreted desoxyribonuclease I that carries the Cys173Ala amino acid substitution, and that is fused to the nucleotide sequence encoding the α-factor signal peptide from Saccharomyces cerevisiae. "AOX1-Prom" denotes the Pichia pastoris AOX1 promoter, "Term" denotes the Pichia pastoris AOX1 terminator. Other pDNM#-2 derivatives described in Example 8 differed with respect to the amino acid substitution encoded in the synthetic DNA sequence encoding the respective variant of bovine secreted desoxyribonuclease I. The corresponding pDNM#-4 vectors that are also derived from pPIC9K lack the α-factor signal peptide from Saccharomyces cerevisiae (Sfu I - Xho I fragment) but instead have the bovine signal peptide of the native bovine pancreatic desoxyribonuclease I pre-protein described in Example 1 inserted at the same position.

## Example 1

### Cloning of the nucleotide sequence encoding the bovine signal peptide of the native bovine pancreatic desoxyribonuclease I pre-protein

Generally, the methods suggested and described in the Invitrogen manuals "Pichia Expression Kit" Version M 011102 25-0043, "pPICZ A, B, and C" Version D 110801 25-0148, "pPICZα A, B, and C" Version E 010302 25-0150, and "pPIC9K" Version E 030402 25-0106 were applied. Reference is also made to further vectors, yeast strains and media mentioned therein. Basic methods of molecular biology were applied as described in Sambrook, Fritsch & Maniatis, Molecular Cloning, A Laboratory Manual, 3rd edition, CSHL Press, 2001.

In order to provide a nucleotide sequence encoding the native bovine signal peptide of the bovine pancreatic desoxyribonuclease I pre-protein, two complementary single-stranded DNA oligonucleotides were synthesised. The base tripletts encoding the native bovine signal peptide, i.e. the codons were designed according to the preferred codon usage in methylotrophic yeast. The DNA oligonucleotides used are given in SEQ ID NO: 39 and SEQ ID NO: 40. The 5' ends of the DNA oligonucleotides were designed such that the annealed, i.e. double-stranded DNA oligonucleotides would have terminal overhangs identical to the overhangs which would have been created by cleavage of restriction endonucleases Sfu I and Xho I. The orientation of the overhangs is given with respect to the coding strand with the Sfu I site being located at its 5' end and the Xho I site being located at its 3' end. Upstream of the coding sequence an optimal Kosak-sequence has been inserted, to facilitate efficient initiation of translation in the host organism.

Of each of the two DNA oligonucleotides 5 μg were dissolved in 10 mM TrisHCl pH 7.5, 10 mM $MgCl_2$, 50 mM NaCl, 1 mM Dithiothreitol and heated at 100°C for 5 minutes, so that unwanted secondary structures and irregular hybridisation products were broken up. Subsequently, hybridisation was allowed to take place by slowly cooling the mixture to room temperature. The double-stranded nucleic acid was analysed in an agarose gel and directly used in a ligation reaction with the expression vector pPICZA (Invitrogen, Carlsbad, CA, USA) which was linearised before with Sfu I and Xho I. The resulting vector which carried the nucleotide sequence encoding the bovine signal peptide of the native bovine pancreatic desoxyribonuclease I pre-protein was subsequently analysed by restriction enzyme digestion and agarose gel electrophoresis as well as by sequencing.

## Example 2

### Mutagenesis of the synthetic nucleotide sequence that encodes bovine pancreatic desoxyribonuclease I

Generally, standard methods of molecular biology were applied as described in Sambrook, Fritsch & Maniatis, Molecular Cloning, A Laboratory Manual, 3rd edition, CSHL Press, 2001. The method explained below is a specific application of a very general method that is also known as "site-directed mutagenesis".

Mutations were generated in a site-directed fashion using the polymerase chain reaction (PCR). In order to mutate a desired codon, i.e. a base triplett, a pair of complementary single-stranded DNA oligonucleotides representing a variant portion of the synthetic nucleotide sequence that encodes bovine pancreatic desoxyribonuclease I were designed and synthesised. The single-stranded DNA oligonucleotides were identical or complementary to the sequence given in SEQ ID NO: 1 except for the triplett sequence to be mutated. Typically, a DNA oligonucleotides had a length of about 20 to 45 nucleotides; the triplett sequence to be mutated or its complement was located in the central portion of the DNA oligonucleotide comprising it, and was flanked on both sides by about 10 to 12 nucleotides. The DNA oligonucleotides were designed such that hybridisation of the DNA oligonucleotides to the wild-type bovine pancreatic desoxyribonuclease I DNA (according to SEQ ID NO: 1) resulted in hybrids with a central mismatch but with intact base pairing at the flanks of the mismatch, including the 5' and 3' ends of each DNA oligonucleotide.

Additionally, two single-stranded DNA oligonucleotide primers were provided, of which the first one, designated "5' DNase I" (SEQ IN NO: 12) comprised the 5'-terminal 21 nucleotides of SEQ ID NO: 1 and the second, designated "3' DNase I" (SEQ IN NO: 13) comprised the sequence complementary to the 3'-terminal 25 nucleotides of SEQ ID NO: 1. The two primers were designed to comprise restriction endonuclease cleavage sites. Therefore, the first and the second primer were extended and included adjacent sequences that were flanking the synthetic nucleotide sequence of SEQ ID NO: 1. "5' DNase I" contained a Xho I site and "3' DNase I" a Not I site.

A nucleotide sequence that encoded a variant, by way of substitution of an amino acid, of the wild-type mature bovine pancreatic desoxyribonuclease I protein was synthesised by means of several PCR-based steps.

A first and a second PCR was carried out using as a template double-stranded DNA comprising the nucleotide sequence according to SEQ ID NO: 1 that was present as an insert in a vector. The vector sequences flanking the insert were such that during PCR the primers "5' DNase I" and "3' DNase I" matched perfectly when annealed. The first PCR was made using a pair of primers consisting of the "5' DNase I" primer and a first single-stranded DNA oligonucleotide comprising the mutated, i.e. variant triplett sequence, whereby the two primers annealed to opposite template DNA strands. The second PCR was made accordingly, using the "3' DNase I" primer and a second single-stranded DNA oligonucleotide, that was complementary to the first one. As a result, the first and the second PCR generated two intermediate products: A 5' and a 3' portion of a nucleotide sequence encoding a variant of bovine pancreatic desoxyribonuclease I, whereby the 5' portion carried the mutated sequence at its 3' end and, vice versa, the 3' portion carried the mutated sequence at its 5' end.

The resulting two intermediate amplification products were analysed by agarose gel electrophoresis, the desired fragments were excised and DNA was isolated from agarose blocks using the "QIAquick Gel Extraction Kit" (Qiagen, catalogue no. 28704).

A third PCR was carried out subsequently, in order to fuse the two portions. To this end, the two portions were united in a single PCR and five PCR cycles were run. During these cycles a few full-length products were formed, whereby the annealing temperature that was used was calculated for the overlapping sequence of the 5' portion and 3' portion. Subsequently, the primers "5' DNase I" and "3' DNase I" were added and 25 more PCR cycles were run, whereby the annealing temperature used here corresponded to the added primer with the lower melting temperature.

A mutated full-length DNA fragment was subsequently inserted into a cloning vector using the "PCR cloning kit - blunt end" (Roche Diagnostics GmbH, Mannheim; catalogue no. 1 939 645). The DNA fragment was verified by means of restriction enzyme analysis and sequencing. The verified DNA fragment was then excised by means of cleavage with Xho I and Not I and inserted into Pichia pastoris expression vectors that were cleaved with the same restriction enzymes (see Example 2 and Eaxmple 4).

**Cys173Ala**

The base triplett "TGC" found in SEQ ID NO: 1 at position 517-519 was substituted by "GCC". To this end, in a first PCR the DNA oligonucleotide "5' Cys173Ala" (SEQ ID NO: 14) was used as a primer in combination with "3' DNase I", and in a second PCR "3' Cys173Ala" (SEQ ID NO: 15) was used as a primer in combination with "5' DNase I". The isolated intermediate fragments were subsequently used for the third PCR, in order to generate the full-length product.

**Cys101Ala**

The base triplett "TGC" found in SEQ ID NO: 1 at position 301-303 was substituted by "GCC". To this end, in a first PCR the DNA oligonucleotide "5' Cys101Ala" (SEQ ID NO: 16) was used as a primer in combination with "3' DNase I", and in a second PCR "3' Cys101Ala" (SEQ ID NO: 17) was used as a primer in combination with "5' DNase I". The isolated intermediate fragments were subsequently used for the third PCR, in order to generate the full-length product.

**Cys104Ala**

The base triplett "TGT" found in SEQ ID NO: 1 at position 310-312 was substituted by "GCT". To this end, in a first PCR the DNA oligonucleotide "5' Cys104Ala" (SEQ ID NO: 18) was used as a primer in combination with "3' DNase I", and in a second PCR "3' Cys104Ala" (SEQ ID NO: 19) was used as a primer in combination with "5' DNase I". The isolated intermediate fragments were subsequently used for the third PCR, in order to generate the full-length product.

**Lys117Asp**

The base triplett "AAA" found in SEQ ID NO: 1 at position 349-351 was substituted by "GAC". To this end, in a first PCR the DNA oligonucleotide "5' Lys117Asp" (SEQ ID NO: 20) was used as a primer in combination with "3' DNase I", and in a second PCR "3' Lys117Asp" (SEQ ID NO: 21) was used as a primer in combination with "5' DNase I". The isolated intermediate fragments were subsequently used for the third PCR, in order to generate the full-length product.

**Arg185His**

The base triplett "AGA" found in SEQ ID NO: 1 at position 553-555 was substituted by "CAC". To this end, in a first PCR the DNA oligonucleotide "5' Arg185His" (SEQ ID NO: 22) was used as a primer in combination with "3' DNase I", and in a second PCR "3' Arg185His" (SEQ ID NO: 23) was used as a primer in combination with "5' DNase I". The isolated intermediate fragments were subsequently used for the third PCR, in order to generate the full-length product.

**Arg185Ala**

The base triplett "AGA" found in SEQ ID NO: 1 at position 553-555 was substituted by "GCA". To this end, in a first PCR the DNA oligonucleotide "5' Arg185Ala" (SEQ ID NO: 24) was used as a primer in combination with "3' DNase I", and in a second PCR "3' Arg185Ala" (SEQ ID NO: 25) was used as a primer in combination with "5' DNase I". The isolated intermediate fragments were subsequently used for the third PCR, in order to generate the full-length product.

**Arg187His**

The base triplett "AGA" found in SEQ ID NO: 1 at position 558-561 was substituted by "CAC". To this end, in a first PCR the DNA oligonucleotide "5' Arg187His" (SEQ ID NO: 26) was used as a primer in combination with "3' DNase I", and in a second PCR "3' Arg187His" (SEQ ID NO: 27) was used as a primer in combination with "5' DNase I". The isolated intermediate fragments were subsequently used for the third PCR, in order to generate the full-length product.

**Arg187Ala**

The base triplett "AGA" found in SEQ ID NO: 1 at position 558-561 was substituted by "GCA". To this end, in a first PCR the DNA oligonucleotide "5' Arg187His" (SEQ ID NO: 28) was used as a primer in combination with "3' DNase I", and in a second PCR "3' Arg187His" (SEQ ID NO: 29) was used as a primer in combination with "5' DNase I". The isolated intermediate fragments were subsequently used for the third PCR, in order to generate the full-length product.

**Ile3Ser**

The base triplett "ATT" found in SEQ ID NO: 1 at position 7-9 was substituted by "TCT". To this end, in a first PCR the DNA oligonucleotide "5' Ile3Ser" (SEQ ID NO: 30) was used as a primer in combination with "3' DNase I". "5' Ile3Ser" comprises the 5' terminal nucleotide sequence similar to "5' DNase I". Therefore, a full-length product was formed and no second and third PCR was necessary in this case.

**Phe82Asn**

The base triplett "TTC" found in SEQ ID NO: 1 at position 244-246 was substituted by "AAC". To this end, in a first PCR the DNA oligonucleotide "5' Phe82Asn" (SEQ ID NO: 31) was used as a primer in combination with "3' DNase I", and in a second PCR "3' Phe82Asn" (SEQ ID NO: 32) was used as a primer in combination with "5' DNase I". The isolated intermediate fragments were subsequently used for the third PCR, in order to generate the full-length product.

**Phe128Asn**

The base triplett "TTC" found in SEQ ID NO: 1 at position 382-384 was substituted by "AAC". To this end, in a first PCR the DNA oligonucleotide "5' Phe128Asn" (SEQ ID NO: 33) was used as a primer in combination with "3' DNase I", and in a second PCR "3' Phe128Asn" (SEQ ID NO: 34) was used as a primer in combination with "5' DNase I". The isolated intermediate fragments were subsequently used for the third PCR, in order to generate the full-length product.

**Cys101Ala, Cys104Ala double mutant**

The base triplett "TGC" found in SEQ ID NO: 1 at position 301-303 was substituted by "GCC" and the base triplett "TGT" at position 310-312 was substituted by "GCT". To this end, in a first PCR the DNA oligonucleotide "5' Cys101Ala, Cys104Ala" (SEQ ID NO: 35) was used as a primer in combination with "3' DNase I", and in a second PCR "3' Cys101Ala, Cys104Ala" (SEQ ID NO: 36) was used as a primer in combination with "5' DNase I". The isolated intermediate fragments were subsequently used for the third PCR, in order to generate the full-length product.

**Arg185Ala, Arg187His double mutant**

The base triplett "AGA" found in SEQ ID NO: 1 at position 553-555 was substituted by "GCA" and the base triplett "AGA" at position 559-561 was substituted by "CAC". To this end, in a first PCR the DNA oligonucleotide "5' Arg185Ala, Arg187His" (SEQ ID NO: 37) was used as a primer in combination with "3' DNase I", and in a second PCR "3' Arg185Ala, Arg187His" (SEQ ID NO: 38) was used as a primer in combination with "5' DNase I". The isolated intermediate fragments were subsequently used for the third PCR, in order to generate the full-length product.

**Lys117Asp, Arg185Ala, Arg187His triple mutant**

The mutation in Lys117Asp was combined with the double mutant Arg185Ala, Arg187His. A unique Sty I cleavage site is located between the mutated sites. Therefore, the full length products obtained as described above were separately cleaved with Sty I and the fragments that contained the desired mutations were isolated following agarose electrophoresis. The cleaved portions were combined such that the fragments that contained the desired mutations could be ligated. Following a standard ligation reaction, the triple mutant full-length fragment was amplified by means of PCR using the primers "5' DNase I" and"3' DNase I". The triple mutant full-length fragment was subsequently verified by sequencing.

**Example 3**

**Cloning of the artificial DNA encoding a variant bovine pancreatic desoxyribonuclease I in pPICZαA- and pPICZA-derived expression vectors expression vectors**

Generally, the methods suggested and described in the Invitrogen manuals "Pichia Expression Kit" Version M 011102 25-0043, "pPICZ A, B, and C" Version D 110801 25-0148, "pPICZα A, B, and C" Version E 010302 25-0150, and "pPIC9K" Version E 030402 25-0106 were applied. Reference is also made to further vectors, yeast strains and media mentioned therein. Basic methods of molecular biology were applied as described in Sambrook, Fritsch & Maniatis, Molecular Cloning, A Laboratory Manual, 3rd edition, CSHL Press, 2001.

The DNA fragment encoding the variant bovine pancreatic desoxyribonuclease I that was generated from PCR fragments (see Example 2) was excised with Xho I and Not I (Roche Diagnostics GmbH). The fragment was isolated using the "QIAquick Gel Extraction Kit" according to the instructions of the manufacturer.

Case 1: The fragment was ligated into the pPICZA vector that comprised the nucleotide sequence encoding the native bovine signal peptide of the bovine pancreatic desoxyribonuclease I pre-protein. The vector was linearised by cleavage with Xho I and Not I and isolated. Then the DNA fragment encoding the variant bovine pancreatic desoxyribonuclease I was inserted and ligated, thereby fusing in-frame the nucleotide sequence encoding the bovine signal peptide with the nucleotide sequence encoding the variant bovine pancreatic desoxyribonuclease I.

Case 2: The fragment was ligated into the pPICZαA vector, thereby fusing the nucleotide sequence encoding the variant bovine pancreatic desoxyribonuclease I to the nucleotide sequence encoding the α-factor signal peptide from Saccharomyces cerevisiae. Before the ligation reaction, the vector was similarly cleaved with Xho I and Not I, and isolated.

The cloning procedure followed in Case 1 inserted a linker sequence - encoding Leucine-Glutamic acid-Lysine-Arginine - into the reading frame between the bovine signal peptide of the native bovine pancreatic desoxyribonuclease I pre-protein and the sequence encoding the variant protein. The Leucine-Glutamic acid sequence was inserted by virtue of the Xho I site (CTCGAG). The Lysine-Arginine sequence is known to represent a KEX-2 signal peptidase cleavage site, needed to cleave off the signal peptide from the pre-protein in the course of the secretory pathway. The cloning procedure followed in Case 2 inserted the nucleotide sequence encoding the variant bovine pancreatic desoxyribonuclease I directly and in-frame after the nucleotide sequence encoding the α-factor signal peptide from Saccharomyces cerevisiae.

In both cases, the nucleotide sequence encoding the recombinant pre-protein were under the control of the P. pastoris AOX-1 promoter (SEQ IN NO.: 11) which, e.g. in Pichia pastoris, is inducible by methanol.

Construction was accomplished by joining in a total volume of 10 μl 20 ng of linearised vector fragment (in a volume of 1 μl), 100 ng of cleaved PCR fragment (in 3 μl), and incubation overnight at 16°C in the presence of T4 DNA ligase (Roche Diagnostics GmbH) according to the instructions of the manufacturer. 5 μl of the ligation preparation were subsequently used to transform competent E. coli XL1Blue cells (Stratagene), in a total volume of 205 μl. Following incubation on ice for 30 min, cells were heat-shocked at 42°C for 90 sec. Subsequently, cells were transferred into 1 ml LB medium and incubated for 1 h at 37°C to allow for expression of selection markers. Aliquots were plated afterwards on LB plates containing 100 μg/ml Zeocin and incubated for 15 h at 37°C. Resistant clones were picked, plasmids were isolated (Sambrook, Fritsch & Maniatis, Molecular Cloning, A Laboratory Manual, 3rd edition, CSHL Press, 2001) and tested by means of restriction analysis as well as sequence analysis. Construct clones verified to be free of errors and cloning artifacts were selected. Expression vectors harbouring a variant bovine pancreatic desoxyribonuclease I with the α-factor signal peptide from Saccharomyces cerevisiae were designated pDNM#-1, expression vectors harbouring variant bovine pancreatic desoxyribonuclease I with the bovine signal peptide of the native bovine pancreatic desoxyribonuclease I pre-protein were designated pDNM#-3, whereby "#" represented a number designating a mutated nucleotide sequence that encoded a particular variant bovine pancreatic desoxyribonuclease I. Table 1 lists the pDNM expression vectors and the inserts that were comprised.

Table 1

pPICZαA- and pPICZA-derived expression vectors

| *variant* | *designation pPICZαA derivative* | *designation pPICZA derivative* | *variant* | *designation pPICZαA derivative* | *designation pPICZA derivative* |
|---|---|---|---|---|---|
| Cys173Ala | pDNM34-1 | pDNM34-3 | Arg187Ala | pDNM1718-1 | pDNM1718-3 |
| Cys101Ala | pDNM35-1 | pDNM35-3 | Ile3Ser | pDNM1920-1 | pDNM1920-3 |
| Cys104Ala | pDNM36-1 | pDNM36-3 | Phe82Asn | pDNM2122-1 | pDNM2122-3 |
| Lys117Asp | pDNM910-1 | pDNM910-3 | Phe128Asn | pDNM2324-1 | pDNM2324-3 |
| Arg185His | pDNM1112-1 | pDNM1112-3 | Cys101Ala, Cys104Ala double mutant | pDNM78-1 | pDNM78-3 |
| Arg185Ala | pDNM1113-1 | pDNM1113-3 | Arg185Ala, Arg187His double mutant | pDNM1516-1 | pDNM1516-3 |
| Arg187His | pDNM1314-1 | pDNM1314-3 | Lys117Asp, Arg185Ala, Arg187His triple mutant | PDNM916-1 | PDNM916-3 |

## Example 4

### Transformation of Pichia pastoris with pPICZaA- and pPICZA-derived pDNM expression vectors

Generally, the methods suggested and described in the Invitrogen manuals "Pichia Expression Kit" Version M 011102 25-0043, "pPICZ A, B, and C" Version D 110801 25-0148, "pPICZα A, B, and C" Version E 010302 25-0150, and "pPIC9K" Version E 030402 25-0106 were applied. Reference is also made to further vectors, yeast strains and media mentioned therein. Basic methods of molecular biology were applied as described in Sambrook, Fritsch & Maniatis, Molecular Cloning, A Laboratory Manual, 3rd edition, CSHL Press, 2001.

The host strains used were Pichia pastoris X-33, GS115, KM71H and SMD1168 (Invitrogen). Preferred strains were X-33 and KM71H. Transformation was aimed at stably integrating expression constructs into the genome of the host organism.

Initially, 5 ml YPD medium (YPD = yeast peptone dextrose; Invitrogen) was inoculated with a P. pastoris colony and pre-cultured on a shaker overnight at 30°C. To prepare transformation-competent cells, 100 μl of the pre-culture were added as inoculum to 200 ml of fresh YPD medium and grown until an $OD_{600nm}$ of between 1.3 and 1.5 was reached. The cells were centrifuged at 1,500 x g for 5 min and resuspended in 200 ml ice cold (0°C) sterile water. The cells were centrifuged again at 1,500 x g for 5 min and resuspended in 100 ml ice cold sterile water. The cells were centrifuged one more time at 1,500 x g for 5 min and resuspended in 10 ml ice cold 1 M sorbitol (ICN). The cells prepared in this way were kept on ice and used for transformation immediately.

The pPICZαA- and pPICZA-derived pDNM expression vectors as given by Table 1 to be used for transformation were linearised using the Sac I restriction endonuclease (Roche Diagnostics GmbH), precipitated and resuspended in water. Transformation was accomplished by electroporation using a "Gene Pulser II™" (BioRad). For a transformation setting, 80 μl P. pastoris cells in 1 M sorbitol solution were mixed gently with 1 μg of linearised expression vector DNA and transferred into an ice cold cuvette which was then kept on ice for 5 min. Subsequently, the cuvette was transferred into the Gene Pulser. Electroporation parameters were 1 kV, 1 kΩ and 25 μF. Following electroporation, 1 ml 1 M sorbitol solution was added to the cell suspension was subsequently plated onto YPDS plates (YPDS = yeast peptone dextrose sorbitol; Invitrogen) containing 100 μg/ml Zeocin™ (Invitrogen), with 100-150 μl of cell suspension being spread on a single plate. YPDS plates were incubated at 30°C for 2-4 days. Yeast clones were transferred onto gridded minimal dextrose plates. Colonies from these plates were picked and separately resuspended in sterile water. The cells were digested with 17.5 units of lyticase (Roche Diagnostics GmbH) for 1 h at 30°C and afterwards frozen for 10 min at -80°C. By means of PCR, the presence of the expression cassettes of the respective pPICZαA- and pPICZA-derived pDNM expression vector was verified. The term "expression cassette" denotes a nucleotide sequence encoding the variant bovine pancreatic desoxyribonuclease I pre-protein, operably linked to the AOX1 promoter and the AOX1 terminator, whereby the expression cassette is derived from the respective pPICZαA- or pPICZA-derived vector used for transformation. As for vectors containing an expression cassette, the terms "vector" and "expression vector" are synonyms.

Positive clones, i.e. clones that were tested positively for the presence of complete expression cassettes stably integrated into the genome were used for further characterisation of variant bovine pancreatic desoxyribonuclease I expression.

Additionally, control transformations were made with the recipient Pichia pastoris X33 strain using the original pPICZαA vector. Positive clones were obtained and verified in a similar fashion.

## Example 5

### Expression and secretion of variant bovine pancreatic desoxyribonuclease I, analysis of pre-proteins with different signal peptides

Generally, the methods suggested and described in the Invitrogen manuals "Pichia Expression Kit" Version M 011102 25-0043, "pPICZ A, B, and C" Version D 110801 25-0148, "pPICZα A, B, and C" Version E 010302 25-0150, and "pPIC9K" Version E 030402 25-0106 were applied. Reference is also made to further vectors, yeast strains and media mentioned therein. Basic methods of molecular biology were applied as described in Sambrook, Fritsch & Maniatis, Molecular Cloning, A Laboratory Manual, 3rd edition, CSHL Press, 2001.

A set of positive clones (usually 20-30) transformed with a pPICZαA- and pPICZA-derived pDNM expression vector (according to Table 1) were grown as shaking cultures overnight, each in 3 ml BMGY medium (BMGY = buffered glycerol-complex medium; Invitrogen). Afterwards, the $OD_{600nm}$ values of the cultures were determined before they were passaged into shaking flasks, each containing 10 ml BMMY medium (Invitrogen) at pH 3. Pre-cultures were used as inoculum to result each in an $OD_{600nm}$ of 1. The cultures were kept on a shaker at 30°C. In parallel, positive control clones were cultured under the same conditions.

BMMY (BMMY = buffered methanol-complex medium;) medium comprises methanol (Mallinckrodt Baker B.V.) which is an inductor of the AOX-1 promoter that controls transcription of the nucleotide sequence encoding the variant bovine pancreatic desoxyribonuclease I.

Samples of 500 μl were taken from the shaking flask in 24 h intervals over a total time of 72 h. When a sample aliquot was removed, the culture was also fed with 0.5% methanol. Samples of the supernatant growth medium were tested for desoxyribonuclease enzymatic activity.

## Example 6

### Analysis of expression of variant bovine pancreatic desoxyribonuclease I

Of the sample aliquots obtained as described in Example 5 firstly the $OD_{600nm}$ was determined. Subsequently the cells were pelleted by centrifugation and the supernatant was saved. Desoxyribonuclease activity was measured in the undiluted supernatant as well as in a 1:10 dilution.

While control clones transformed with the pPICZαA vector did not lead to any measurable desoxyribonuclease activity in the medium, Pichia strains transformed with pPICZαA- and pPICZA-derived pDNM expression vectors (according to Table 1) showed desoxyribonuclease activity due to the respective variant of bovine pancreatic desoxyribonuclease I secreted into the growth medium, i.e. the culture medium. It could therefore be concluded that expression of a recombinant pre-protein comprising either the α-factor signal peptide from Saccharomyces cerevisiae or the bovine signal peptide of the native bovine pancreatic desoxyribonuclease I pre-protein enables secretion of an active enzyme having desoxyribonuclease activity.

Regarding the yield of secreted protein, i.e. the desired variant of bovine pancreatic desoxyribonuclease I, there were no obvious differences between the strains expressing the pre-protein with the bovine signal peptide of the native bovine pancreatic desoxyribonuclease I pre-protein and the pre-protein with the α-factor signal peptide from Saccharomyces cerevisiae.

## Example 7

### Increasing expression yield by multiple transformation and increased Zeocin™ concentration

Generally, the methods suggested and described in the Invitrogen manuals "Pichia Expression Kit" Version M 011102 25-0043, "pPICZ A, B, and C" Version D 110801 25-0148, "pPICZα A, B, and C" Version E 010302 25-0150, and "pPIC9K" Version E 030402 25-0106 were applied. Reference is also made to further vectors, yeast strains and media mentioned therein. Basic methods of molecular biology were applied as described in Sambrook, Fritsch & Maniatis, Molecular Cloning, A Laboratory Manual, 3rd edition, CSHL Press, 2001.

The yeast clones transformed with the pPICZαA- and pPICZA-derived pDNM expression vectors (according to Table 1) that were found to produce the highest desoxyribonuclease activities in supernatant media were subjected to repeated electroporation using the same expression vector as previously. Conditions for electroporation were as described in Example 4 with the exception that YPDS plates contained Zeocin™ at increased concentrations, that is between 1,000 and 2,000 μg/ml. The concentration of the antibiotic was increased in order to select for transformants having incorporated into their genome multiple copies of the respective expression vector. Yeast clones with increased resistance to the antibiotic were transferred onto gridded minimal dextrose plates. As already described in Example 5, pre-cultures were made from individual yeast clones and expression was measured by determining the desoxyribonuclease enzymatic activity secreted into the growth medium as described in Example 6. Individual clones were found that produced an increased amount of desoxyribonuclease activity. This was the case for yeast transformants expressing both types of recombinant pre-protein, i.e. pre-protein comprising either the α-factor signal peptide from Saccharomyces cerevisiae or the bovine signal peptide of the native bovine pancreatic desoxyribonuclease I pre-protein. On the average, desoxyribonuclease activity measured in the supernatant of Pichia strains repeatedly transformed with the respective pPICZαA- and pPICZA-derived pDNM expression vector was between twice to three times as high compared to the respective precursor strains that had undergone only a single transformation.

Regarding the yield of secreted mature protein, there were no obvious differences between the strains expressing the pre-protein with the bovine signal peptide of the native bovine pancreatic desoxyribonuclease I pre-protein and the pre-protein with the α-factor signal peptide from Saccharomyces cerevisiae.

## Example 8

### Increasing expression yield by means of introducing a different expression vector allowing to apply further selection pressure

Generally, the methods suggested and described in the Invitrogen manuals "Pichia Expression Kit" Version M 011102 25-0043, "pPICZ A, B, and C" Version D 110801 25-0148, "pPICZα A, B, and C" Version E 010302 25-0150, and "pPIC9K" Version E 030402 25-0106 were applied. Reference is also made to further vectors, yeast strains and media mentioned therein. Basic methods of molecular biology were applied as described in Sambrook, Fritsch & Maniatis, Molecular Cloning, A Laboratory Manual, 3rd edition, CSHL Press, 2001.

From each of the pPICZαA- and pPICZA-derived pDNM expression vectors according to Table 1 the respective expression cassette consisting of a part of the AOX-1 promoter and the reading frame for the respective pre-protein was excised using restriction endonucleases Sac I and Xba I (Roche Diagnostics GmbH). The resulting cleavage products were separated by agarose gel electrophoresis. In the isolated fragments with the expression cassette, the Xba I overhang was converted to a blunt end using Klenow polymerase (Roche Diagnostics GmbH).

The vector pPIC9K (Invitrogen) was cleaved using restriction endonucleases Sac I and Not I (Roche Diagnostics GmbH). The resulting cleavage products were separated by agarose gel electrophoresis. A fragment with a size of 8956 bp was excised and isolated using the "QIAquick Gel Extraction Kit" (Qiagen). The Not I overhang was converted to a blunt end using Klenow polymerase (Roche Diagnostics GmbH). The expression cassettes prepared from the pPICZαA- and pPICZA-derived pDNM expression vectors according to Table 1 were inserted separately. Ligation, bacterial transformation and cloning procedures were performed as described in Example 3 with the exception that transformed bacterial clones were selected on LB plates containing 100 μg/ml of the antibiotic ampicillin. Clones were verified by means of restriction analysis and sequencing. The pPIC9K-derived expression vectors harbouring the variant of bovine pancreatic desoxyribonuclease I with the α-factor signal peptide from Saccharomyces cerevisiae were designated pDNM#-2, the pPIC9K-derived expression vector harbouring the variant of bovine pancreatic desoxyribonuclease I with the bovine signal peptide of the native bovine pancreatic desoxyribonuclease I pre-protein were designated pDNM#-4, whereby "#" represented a number designating a mutated nucleotide sequence that encoded a particular variant bovine pancreatic desoxyribonuclease I. Table 2 lists the pDNM expression vectors and the inserts that were comprised.

Table 2

pPIC9K-derived expression vectors

| *variant* | *designation pPIC9K derivative, α-factor signal peptide* | *designation pPIC9K derivative, bovine signal peptide* | *variant* | *designation pPIC9K derivative, α-factor signal peptide* | *designation pPIC9K derivative, bovine signal peptide* |
|---|---|---|---|---|---|
| Cys173Ala | pDNM34-2 | pDNM34-4 | Arg187Ala | pDNM1718-2 | pDNM1718-4 |
| Cys101Ala | pDNM35-2 | pDNM35-4 | Ile3Ser | pDNM1920-2 | pDNM1920-4 |
| Cys104Ala | pDNM36-2 | pDNM36-4 | Phe82Asn | pDNM2122-2 | pDNM2122-4 |
| Lys117Asp | pDNM910-2 | pDNM910-4 | Phe128Asn | pDNM2324-2 | pDNM2324-4 |
| Arg185His | pDNM1112-2 | pDNM1112-4 | Cys101Ala, Cys104Ala double mutant | pDNM78-2 | pDNM78-4 |
| Arg185Ala | pDNM1113-2 | pDNM1113-4 | Arg185Ala, Arg187His double mutant | pDNM1516-2 | pDNM1516-4 |
| Arg187His | pDNM1314-2 | pDNM1314-4 | Lys117Asp, Arg185Ala, Arg187His triple mutant | PDNM916-2 | PDNM916-4 |

Using the pPIC9K-derived expression vectors, resistance to the antibiotic G418 was introduced. Among the Pichia pastoris Zeocin™ -resistant transformants having incorporated into their genome multiple copies of the pPICZαA- or pPICZA-derived pDNM expression vectors those were selected that secreted into the growth medium the highest amounts of desoxyribonuclease activity. Clones containing multiple copies of a given pPICZαA- or pPICZA-derived pDNM expression vector were transformed with a pPIC9K-derived expression vector that carried the same expression cassette. As before, pPIC9K-derived expression vectors to be used for transformation were linearised, using the Sal I restriction endonuclease (Roche Diagnostics GmbH). 1 μg of the respective linearised expression vector was used for transformation which was performed as described in Example 4. Following electroporation, the cells were kept at 4°C in 1 M sorbitol for a period of between 1 and 3 days, in order to allow the cells become resistant to the antibiotic. The cell suspension was plated onto YPDS plates (Invitrogen) containing 1, 2 and 4 mg/ml G418 (Roche Diagnostics GmbH), with 100-200 μl of cell suspension being spread on a single plate. YPDS plates were incubated at 30°C for 3-5 days. Yeast clones were transferred onto gridded minimal dextrose plates. Clones originating from YPDS plates with the highest G418 concentration were preferentially transferred. Selected clones were characterised further as described in Example 4.

Multiply transformed and verified Pichia clones carrying multiple copies of expression vectors conferring Zeocin™ resistance as well as the expression vector conferring resistance to G418 were characterised with respect to the amount of desoxyribonuclease activity secreted into the growth medium. Assays were performed as described in Example 5. Multiply transformed clones carrying expression both, a pPICZαA- or pPICZA-derived pDNM expression vector and a pPIC9K-derived expression vector, were identified which produced an even higher level of secreted desoxyribonuclease enzymatic activity than the precursor clones. On the average, desoxyribonuclease activity measured in the supernatant of cultures that were transformed with both, a pPICZαA- or pPICZA-derived pDNM expression vector and a pPIC9K-derived expression vector, was found to be about four times as high when compared to the respective precursor strains that had undergone only a single transformation.

Regarding the yield of the secreted variant of bovine pancreatic desoxyribonuclease I, there were no obvious differences between the strains expressing the pre-protein comprising the bovine signal peptide of the native bovine pancreatic desoxyribonuclease I pre-protein and the pre-protein comprising the α-factor signal peptide from Saccharomyces cerevisiae.

**Example 9**

**Purification of variant bovine pancreatic desoxyribonuclease I from liquid culture supernatant**

Biomass was removed from the supernatant growth medium by filtration or by centrifugation. Variant bovine pancreatic desoxyribonuclease I was subsequently purified by means of ion exchange chromatography using a cation exchanger. Binding to the cation exchanger took place using a binding buffer that had a pH of 5.0 and contained 20 mM $Ca^{2+}$ acetate. Other proteins and impurities were removed by washing the solid phase repeatedly with binding buffer, whereby the variant bovine pancreatic desoxyribonuclease I remained bound by the solid phase, that is the cation exchanger. Elution of the variant of bovine pancreatic desoxyribonuclease I was accomplished using an elution buffer that had a pH of 5.0 and contained 0.3 M NaCl, 20 mM $Ca^{2+}$ acetate. The purity of the variant bovine pancreatic desoxyribonuclease I achieved after this step was higher than about 95% as tested by means of SDS PAGE, whereby gels were stained using Coomassie Blue. The subsequent purification step was affinity chromatography using heparin sepharose. Following this step, the purity of the variant of bovine pancreatic desoxyribonuclease I was higher than about 98% as tested by means of SDS PAGE, whereby gels were stained using Coomassie Blue.

**Example 10**

**Assay to determine the specific desoxyribonuclease activity of variant bovine pancreatic desoxyribonuclease I in growth culture supernatant**

The test for desoxyribonuclease activity in sample aliquots was performed according to Kunitz (Kunitz, M., J. Gen. Physiol. 33 (1950) 349-62 and 363). Calf thymus DNA was dissolved at a concentration of 0.05 mg/ml in a buffer containing 10 mM TrisHCl pH 8.0, 0.1 mM $CaCl_2$, 1 mM $MgCl_2$. Desoxyribonuclease activity-containing growth medium such as cleared (filtrated) culture supernatant was added and the increase of the extinction at 260 nm was photometrically measured over time at 25°C. 1 unit (1U) corresponds to an extinction increase ($\Delta$E) of 0.001 per min.

**Example 11**

**Assay to determine the specific desoxyribonuclease activity of purified variant bovine pancreatic desoxyribonuclease I**

The desoxyribonuclease-free reference sample was the sample buffer, that is a mixture of 1 part 1 M sodium acetate pH 5.0, 1 part 50 mM $MgSO_4$ and 8 parts double-distilled water. For the substrate buffer, calf thymus DNA was dissolved in a buffer containing 5 mM $MgSO_4$ and 100 mM sodium acetate pH 5.0 and incubated between 24 to 30 hours in a water bath at 37°C. Unsoluble parts were removed by centrifugation for 10 min at 13,000 x g. Substrate buffer contained DNA at a concentration of 0.04 mg/ml. DNA content of the supernatant was determined photometrically at 260 nm and, if necessary, the substrate buffer was adjusted with sample buffer to give an extinction value of 0.8. Substrate buffer was stored for at least 3 days at 4°C before use.

In an exemplary measurement, desoxyribonuclease-containing solution with a volume activity of about 1,000 units per ml obtained from purification of variant bovine pancreatic desoxyribonuclease I according to Example 9 was used for the determination of desoxyribonuclease activity. 60 $\mu$l of the desoxyribonuclease-containing solution was diluted with 40 $\mu$l double-distilled water (in case a sample with another volume activity was measured, the dilution ratio was adjusted). Firstly, 2.5 ml substrate buffer was filled into a quartz cuvette with a thickness of 1 cm. Both the substrate buffer and the cuvette were kept at 25°C, measurements were at the same temperature. The wave length at which measurements were taken was 260 nm. After the photometer was set to zero extinction (reference value) 0.05 ml diluted desoxyribonuclease-containing solution was added and mixed. The increase of the extinction ($\Delta$E/min) was measured over time. One unit (1 U) corresponds to the activity that under the conditions as described above leads to an increase of the extinction of 0.001 per min.

The activity per volume given as was calculated as

$$[U / ml] = \frac{2{,}55 \text{ x } 1{,}000 \text{ x } \Delta E/min}{0.05}$$

The activity of undiluted variant bovine pancreatic desoxyribonuclease I preparations was calculated according to the dilution factor applied. It was also generally observed that the units measured using this assay were comparable to those of the Kunitz assay.

Additionally, protein content was measured using the same type of cuvettes as above. Measurements were taken of purified variant bovine pancreatic desoxyribonuclease I in sample buffer at temperatures between 20°C and 25°C, at a wave length of 280 nm, with the sample buffer serving as reference.

The protein content was calculated from extinction values ($\Delta E_{280}$) as

$$[\text{mg protein / ml}] = \Delta E_{280} \text{ x } 0.796$$

Each measurement was taken in triplicate. Specific desoxyribonuclease activity in a given volume was then calculated as units per mg of protein.

## Example 12

**Assay to determine the specific desoxyribonuclease activity following heat incubation**

Aliquots of the purified variant of bovine pancreatic desoxyribonuclease I in a storage buffer containing 20 mM TrisHCl, 2 mM $MgCl_2$, 4 mM $CaCl_2$, 50% glycerol, pH 7.6 were incubated for 5 min at 94°C, whereby each aliquot had a volume of 500 μl and contained 500 or more units per ml (as determined by the assay in Example 11). Each aliquot was kept over the heating period in a fine-regulated (variation limit less than 0.5°C) thermostate block heater. Immediately after the incubation, residual specific desoxyribonuclease activity was measured as activity per volume using the assay as described in Example 11.

Test results were obtained with the following variants: Arg185Ala; Arg185His; Arg187A1a; Arg187His; Arg185Ala, Arg187His double mutant; Lys117Asp, Arg185Ala, Arg187His triple mutant; Cys101Ala; Cys104Ala; Cys101Ala, Cys104Ala double mutant; Cys173Ala; Ile3Ser; Lys117Asp; Phe128Asn; and Phe82Asn. Residual activity per volume after heat treatment, that is 94°C for 5 min, was zero units per mg of protein. No differences regarding heat stability were found with respect to Pichia yeast strains used for transformation or the kind of signal peptide that was comprised in the respective pre-protein.

The triple mutant Lys117Asp, Arg185A1a, Arg187His was selected and subjected for further testing. Enzyme concentrations and assay conditions were as described above with the exception that heat treatment was 70°C for 5 min. Following the heat treatment, the specific desoxyribonuclease activity was zero units per mg of protein. No differences regarding heat stability were found with respect to Pichia yeast strains used for transformation or the kind of signal peptide that was comprised in the respective pre-protein. After the heat treatment, loss of desoxyribonuclease activity could not be reversed, e.g. by a lower temperature or an increased concentration of $Ca^{2+}$ or $Mg^{2+}$ ions.

## Example 13

**Assay to determine ribonuclease activity of the Lys117Asp, Arg185Ala, Arg187His triple mutant variant of bovine pancreatic desoxyribonuclease I**

In a total volume of 50 μl containing 5 μl 10x "Shure Cut Buffer L" (Roche Diagnostics GmbH, Mannheim; Cat. No. 1417975) and 5 μg purified bacteriophage MS2 RNA " (Roche Diagnostics GmbH, Mannheim; Cat. No. 0165948), 10 units of the Lys117Asp, Arg185Ala, Arg187His triple mutant variant of bovine pancreatic desoxyribonuclease I were incubated for 4 h at 37°C. At a 1x concentration the "Shure Cut Buffer L" contains 10 mM Tris pH 7.5, 10 mM $MgCl_2$, and 1 mM dithioerythritol.

Following agarose gel electrophoresis of a 20 μl aliquot of the reaction mix in a 2% agarose gel, no RNA degradation could be detected when compared with the untreated control RNA.

## List of References

Alberts, B., Johnson, A., Lewis, J., Raff, M., Roberts, K., Walter, P. (eds), Molecular Biology of the Cell, fourth edition, 2002, Garland Science Publishing

Beck, E., et al., Gene 19 (1982) 327-336

Bickler et al., Biotechniques 13 (1992) 64-66

Carmels, T., et al., Curr. Genet. 20 (1991) 309-314
Chen, X.J., and Fukuhara, H., Gene (1988) 181-192
Drocourt, D., et al., Nucleic Acids Res. 18 (1990) 4009
EP 0 116 201
Funakoshi, A., et al., J. Biochem. (Tokyo) 88 (1980) 1113-1138
Hanaki K. et al. Biotechniques 29 (2000) 38-42
Julius, D., et al., Cell 37 (1984) 1075-1089
Kunitz, M., J. Gen. Physiol. 33 (1950) 349-62 and 363
Lahm, A. & Suck, D., J. Mol. Biol. 221 (1991) 645-667
Nefsky, B., and Bretscher, A., Eur. J. Biochem. 179 (1989) 215-219
Paudel, H.K., and Liao, T.H., J. Biol Chem. 261 (1986) 16006-16011
Sambrook, Fritsch & Maniatis, Molecular Cloning, A Laboratory Manual, 3rd edition, CSHL Press, 2001
Southern, P.J., and Berg, P., J. Mol. Appl. Genet. 1 (1982) 327-341
Suck, D. et al., Nature 332 (1988) 464-468
Thill, G.P., et al., Positive and Negative Effects of Multi-Copy Integrated Expression in Pichia pastoris, International Symposium on the Genentics of Microorganisms 2 (1990), pp. 477-490
US 2002/0042052 A1
US 4,683,293
US 4,808,537
US 4,812,405
US 4,818,700
US 4,837,148
US 4,855,231
US 4,857,467
US 4,870,008
US 4,879,231
US 4,882,279
US 4,885,242
US 4,895,800
US 4,929,555
US 5,002,876
US 5,004,688
US 5,032,516
US 5,122,465
US 5,135,868
US 5,166,329
US 5,324,639
US 5,618,676
US 5,854,018
US 5,856,123
US 5,919,651
van Treeck, U., et al., Antimicrob Agents Chemother. 19 (1981) 371-380
Vedvick, T., et al., J. Ind. Microbiol. 7 (1991) 197-201
Wang W.-Y. et al. Biochem J. 346 (2000) 799-804
Waters et al., J. Biol. Chem. 263 (1988) 6209-14
Werten, M.W., et al., Yeast 15 (1999) 1087-1096
WO 00/56903

## Claims

1. A variant, by way of amino acid substitution, of bovine pancreatic desoxyribonuclease I, wherein at least one different amino acid substitutes for an amino acid residue, that is at least one of the amino acid residues of bovine pancreatic desoxyribonuclease I selected from the group consisting of
Cys173, Cys101, Cys104, Lys117, Arg185, Arg187, Ile3, Phe82, and Phe128,
numbered from the N-terminus of the 260-amino acid bovine pancreatic desoxyribonuclease I according to SEQ ID NO: 2 (wild-type reference), to form a bovine pancreatic desoxyribonuclease I variant with desoxyribonuclease activity and increased thermolability compared to the wild-type reference, whereby the different amino acid is selected from the group consisting of

    - Ala, Ser, Thr, Gly, and Val when the different amino acid substitutes for a Cys173, Cys 101 or Cys 104 residue,
    - Asp, Glu, Asn, Gln, and Ile when the different amino acid substitutes for the Lys 117 residue,
    - His, Ala, Asn, and Gln when the different amino acid substitutes for an Arg185 or Arg187 residue,
    - Ala, Ser, Thr, Gly, and Val when the different amino acid substitutes for the Ile3 residue,
    - Asn, Gln, and Ile when the different amino acid substitutes for a Phe82 or Phe128 residue.

2. The variant of bovine pancreatic desoxyribonuclease I according to claim 1, **characterised in that**
the specific desoxyribonuclease activity of the variant of bovine pancreatic desoxyribonuclease I is approximately zero units per mg of protein following heating of the variant of bovine pancreatic desoxyribonuclease I for about 5 min at a temperature less than 95°C.

3. A method to produce a variant of bovine pancreatic desoxyribonuclease I according to any of the claims 1 and 2, comprising the steps of

    (a) providing a vector comprising a nucleotide sequence that encodes the variant of bovine pancreatic desoxyribonuclease I,
    (b) transforming a microbial host strain with the vector,
    (c) cultivating the transformed microbial host strain in a growth medium that contains nutrients, whereby the microbial host strain expresses the variant of bovine pancreatic desoxyribonuclease I, and
    (d) purifying the variant of bovine pancreatic desoxyribonuclease I from the microbial host strain and/or the growth medium.

4. The method according to claim 3, **characterised in that**
the nucleotide sequence that encodes the variant of bovine pancreatic desoxyribonuclease I is SEQ ID NO: 3.

5. The method according to any of the claims 3 and 4, **characterised in that**

    (a) the vector comprises a nucleotide sequence that encodes a pre-protein consisting of the variant of bovine pancreatic desoxyribonuclease I and a signal peptide,
    (b) the microbial host strain is a methylotrophic yeast strain,
    (c) the growth medium contains methanol as a carbon source,
    (d) the methylotrophic yeast strain expresses and secretes the variant of bovine pancreatic desoxyribonuclease I, and
    (e) the variant of bovine pancreatic desoxyribonuclease I is purified from the growth medium.

6. The method according to claim 5, **characterised in that**
the signal peptide contains a signal peptidase cleavage site which is located directly adjacent to the first amino acid of the variant of bovine pancreatic desoxyribonuclease I.

7. The method according to any of the claims 5 and 6, **characterised in that**
the amino acid sequence of the expressed pre-protein is selected from the group consisting of

    (a) SEQ ID NO: 8,
    (b) SEQ ID NO: 9, and
    (c) SEQ ID NO: 10.

8. The method according to any of the claims 5 to 7, **characterised in that**

the nucleotide sequence encoding the variant of bovine pancreatic desoxyribonuclease I is SEQ ID NO: 3.

**9.** The method according to any of the claims 5 to 8, **characterised in that** the nucleotide sequence encoding the pre-protein consists of the nucleotide sequence encoding the signal peptide fused to the nucleotide sequence encoding the variant of bovine pancreatic desoxyribonuclease I.

**10.** The method according to any of the claims 5 to 9, **characterised in that** the nucleotide sequence encoding the signal peptide is selected from the group consisting of

(a) SEQ ID NO: 5,
(b) SEQ ID NO: 6, and
(c) SEQ ID NO: 7.

**11.** The method according to any of the claims 5 to 10, **characterised in that** the nucleotide sequence encoding the pre-protein is operably linked to a promoter or promoter element.

**12.** The method according to any of the claims 5 to 11, **characterised in that** the methylotrophic yeast strain is a Hansenula, Pichia, Candida or Torulopsis species.

**13.** The method according to claim 12, **characterised in that** the methylotrophic yeast strain is selected from the group consisting of Pichia pastoris, Hansenula polymorpha, Candida boidinii and Torulopsis glabrata.

**14.** The method according to claim 13, **characterised in that** the methylotrophic yeast strain is the Pichia pastoris strain with the American Type Culture Collection accesssion number 76273 or a derivative thereof.

**15.** A Pichia pastoris strain with a chromosome that contains a vector comprising a nucleotide sequence that encodes a pre-protein consisting of the variant of bovine pancreatic desoxyribonuclease I and a signal peptide, operably linked with the Pichia pastoris AOX1 promoter according to SEQ ID NO: 11 or a promoter element thereof,
whereby the nucleotide sequence that encodes the pre-protein is SEQ ID NO: 6 or SEQ ID NO: 7, fused to SEQ ID NO: 3.

**16.** Use of a variant of bovine pancreatic desoxyribonuclease I according to any of the claims 1 and 2
for hydrolysing DNA and subsequently reducing the specific desoxyribonuclease activity of the variant of bovine pancreatic desoxyribonuclease I to approximately zero units per mg of protein by heating of the variant of bovine pancreatic desoxyribonuclease I for about 5 min at a temperature less than 95°C.

**17.** Use of a variant of bovine pancreatic desoxyribonuclease I according to claim 16, **characterised in that** the specific desoxyribonuclease activity of the variant of bovine pancreatic desoxyribonuclease I is reduced to approximately zero units per mg of protein by heating of the variant of bovine pancreatic desoxyribonuclease I for about 5 min at a temperature of approximately 70°C.

**18.** Kit of parts containing the variant of bovine pancreatic desoxyribonuclease I according to any of the claims 1 and 2 and a reaction buffer comprising a divalent cation.

**19.** Kit of parts according to claim 18, **characterised in that** the variant of bovine pancreatic desoxyribonuclease I is dissolved in a buffer containing 2 mM TrisHCl, 2 mM $MgCl_2$, 4 mM $CaCl_2$, 50% glycerol, pH 7.6, and the ten times concentrated reaction buffer contains 100 mM TrisHCl pH 7.5, 100 mM MgCl2, and 10 mM dithioerythritol.

**Patentansprüche**

**1.** Variante durch Aminosäuresubstitution von Desoxyribonuklease I aus Rinderpankreas, wobei mindestens eine andere Aminosäure anstelle eines Aminosäurerests eingesetzt wird, der zumindest einer der Aminosäurereste von Desoxyribonuklease I aus Rinderpankreas ist, ausgewählt aus der Gruppe, bestehend aus
Cys173, Cys101, Cys104, Lys117, Arg185, Arg187, Ile3, Phe82 und Phe128,

die vom N-Terminus der 260 Aminosäuren langen Desoxyribonuklease I aus Rinderpankreas gemäß SEQ ID NR: 2 (Wildtyp-Referenz) nummeriert sind, so dass eine Variante von Desoxyribonuklease I aus Rinderpankreas mit Desoxyribonuklease-Aktivität und erhöhter Thermolabilität gegenüber der Wildtyp-Referenz erhalten wird, wobei die andere Aminosäure ausgewählt ist aus der Gruppe, bestehend aus:

- Ala, Ser, Thr, Gly und Val, wenn die andere Aminosäure anstelle eines Cys173-, Cys101- oder Cys104-Rests eingesetzt wird,
- Asp, Glu, Asn, Gln und Ile, wenn die andere Aminosäure anstelle eines Lys117-Rests-eingesetzt wird,
- His, Ala, Asn und Gln, wenn die andere Aminosäure anstelle eines Arg185- oder Arg187-Rests eingesetzt wird,
- Ala, Ser, Thr, Gly und Val, wenn die andere Aminosäure anstelle eines Ile3-Rests eingesetzt wird,
- Asn, Gln und Ile, wenn die andere Aminosäure anstelle eines Phe82- oder Phe128-Rests eingesetzt wird.

2. Variante von Desoxyribonuklease I aus Rinderpankreas nach Anspruch 1, **dadurch gekennzeichnet, dass** die spezifische Desoxyribonuklease-Aktivität der Variante von Desoxyribonuklease I aus Rinderpankreas nach dem Erhitzen der Variante von Desoxyribonuklease I aus Rinderpankreas für etwa 5 min bei einer Temperatur von weniger als 95°C null Einheiten pro mg Protein ausmacht.

3. Verfahren zur Herstellung einer Variante von Desoxyribonuklease I aus Rinderpankreas nach einem der Ansprüche 1 und 2, umfassend die Schritte

(a) Bereitstellen eines Vektors, umfassend eine Nukleotidsequenz, die die Variante von Desoxyribonuklease I aus Rinderpankreas codiert,
(b) Transformieren eines mikrobiellen Wirtsstammes mit dem Vektor,
(c) Züchten des transformierten mikrobiellen Wirtsstammes in einem Wachstumsmedium, das Nährstoffe enthält, wodurch der mikrobielle Wirtsstamm die Variante von Desoxyribonuklease I aus Rinderpankreas exprimiert, und
(d) Reinigen der Variante von Desoxyribonuklease I aus Rinderpankreas aus dem mikrobiellen Wirtsstamm und/oder dem Wachstumsmedium.

4. Verfahren nach Anspruch 3, **dadurch gekennzeichnet, dass** die Nukleotidsequenz, die die Variante von Desoxyribonuklease I aus Rinderpankreas codiert, SEQ ID NR: 3 ist.

5. Verfahren nach einem der Ansprüche 3 und 4, **dadurch gekennzeichnet, dass**

(a) der Vektor eine Nukleotidsequenz umfasst, die ein Präprotein codiert, bestehend aus der Variante von Desoxyribonuklease I aus Rinderpankreas und einem Signalpeptid,
(b) der mikrobielle Wirtsstamm ein methylotropher Hefestamm ist,
(c) das Wachstumsmedium Methanol als Kohlenstoffquelle enthält,
(d) der methylotrophe Hefestamm die Variante von Desoxyribonuklease I aus Rinderpankreas exprimiert und sezerniert, und
(e) die Variante von Desoxyribonuklease I aus Rinderpankreas aus dem Wachstumsmedium gereinigt wird.

6. Verfahren nach Anspruch 5, **dadurch gekennzeichnet, dass** das Signalpeptid eine Signalpeptidase-Spaltstelle enthält, die sich direkt neben der ersten Aminosäure der Variante von Desoxyribonuklease I aus Rinderpankreas befindet.

7. Verfahren nach einem der Ansprüche 5 und 6, **dadurch gekennzeichnet, dass** die Aminosäuresequenz des exprimierten Präproteins ausgewählt ist aus der Gruppe, bestehend aus

(a) SEQ ID NR: 8,
(b) SEQ ID NR: 9, und
(c) SEQ ID NR: 10.

8. Verfahren nach einem der Ansprüche 5 bis 7, **dadurch gekennzeichnet, dass** die Nukleotidsequenz, die die Variante von Desoxyribonuklease I aus Rinderpankreas codiert, SEQ ID NR: 3 ist.

9. Verfahren nach einem der Ansprüche 5 bis 8, **dadurch gekennzeichnet, dass** die das Präprotein codierende Nukleotidsequenz aus der das Signalpeptid codierenden Nukleotidsequenz besteht, die an die Nukleotidsequenz gebunden ist, die die Variante der Desoxyribonuklease I aus Rinderpankreas codiert.

**10.** Verfahren nach einem der Ansprüche 5 bis 9, **dadurch gekennzeichnet, dass** die das Signalpeptid codierende Nukleotidsequenz ausgewählt ist aus der Gruppe, bestehend aus

(a) SEQ ID NR: 5,
(b) SEQ ID NR: 6 und
(c) SEQ ID NR: 7.

**11.** Verfahren nach einem der Ansprüche 5 bis 10, **dadurch gekennzeichnet, dass** die das Präprotein codierende Nukleotidsequenz funktionsfähig an einen Promotor oder ein Promotorelement gebunden ist.

**12.** Verfahren nach einem der Ansprüche 5 bis 11, **dadurch gekennzeichnet, dass** der methylotrophe Hefestamm eine Hansenula-, Pichia-, Candida- oder Torulopsis-Spezies ist.

**13.** Verfahren nach Anspruch 12, **dadurch gekennzeichnet, dass** der methylotrophe Hefestamm ausgewählt ist aus der Gruppe, bestehend aus Pichia pastoris, Hansenula polymorpha, Candida boidinii und Torulopsis glabrata.

**14.** Verfahren nach Anspruch 13, **dadurch gekennzeichnet, dass** der methylotrophe Hefestamm der Pichia-pastoris-Stamm mit der Zugangs-Nummer 76273 bei der American Type Culture Collection oder ein Derivat davon ist.

**15.** Pichia-pastoris-Stamm mit einem Chromosom, das einen Vektor enthält, umfassend eine Nukleotidsequenz, die ein aus der Variante von Desoxyribonuklease I aus Rinderpankreas und einem Signalpeptid bestehendes Präprotein codiert, und die funktionsfähig an den Pichia-pastoris-AOX1-Promoter der SEQ ID NR: 11 oder ein Promotorelement davon gebunden ist,
wobei die das Präprotein codierende Nukleotidsequenz die an SEQ ID NR: 3 gebundene SEQ ID NR: 6 oder SEQ ID NR: 7 ist.

**16.** Verwendung einer Desoxyribonuklease I aus Rinderpankreas nach einem der Ansprüche 1 und 2 zur Hydrolyse von DNA und anschließendes Reduzieren der spezifischen Desoxyribonuklease-Akivität der Variante von Desoxyribonuklease I aus Rinderpankreas auf etwa null Einheiten pro mg Protein durch Erhitzen der Variante der Desoxyribonuklease I aus Rinderpankreas für etwa 5 min bei einer Temperatur von weniger als 95°C.

**17.** Verwendung einer Desoxyribonuklease I aus Rinderpankreas nach Anspruch 16, **dadurch gekennzeichnet, dass** die spezifische Desoxyribonuklease-Aktivität der Variante von Desoxyribonuklease I aus Rinderpankreas durch Erhitzen der Variante der Desoxyribonuklease I aus Rinderpankreas für etwa 5 min bei einer Temperatur von etwa 70°C auf etwa null Einheiten pro mg Protein reduziert wird.

**18.** Teile-Kit, enthaltend die Variante von Desoxyribonuklease I aus Rinderpankreas nach einem der Ansprüche 1 und 2 und einen Reaktionspuffer, der ein zweiwertiges Kation umfasst.

**19.** Teile-Kit nach Anspruch 18, **dadurch gekennzeichnet, dass** die Variante von Desoxyribonuklease I aus Rinderpankreas in einem Puffer gelöst ist, der 2 mM Tris-HCl, 2 mM $MgCl_2$, 4 mM $CaCl_2$, 50% Glycerin, pH-Wert 7,6, enthält, und der 10-fach konzentrierte Reaktionspuffer 100 mM Tris-HCl, pH-Wert 7,5, 100 mM $MgCl_2$ und 10 mM Dithioerythritol enthält.

**Revendications**

**1.** Variant, au moyen d'une substitution d'acide aminé, d'une désoxyribonucléase I pancréatique bovine, dans lequel au moins un acide aminé différent remplace un résidu d'acide aminé, qui est au moins l'un des résidus d'acides aminés de la désoxyribonucléase I pancréatique bovine choisi dans le groupe constitué par
la Cys173, la Cys101, la Cys104, la Lys117, l'Arg185, l'Arg187, l'Ile3, la Phe82, et la Phe128,
numérotés de l'extrémité N de l'acide aminé 260 de la désoxyribonucléase I pancréatique bovine selon SEQ ID NO: 2 (référence de type sauvage), pour former un variant de désoxyribonucléase I pancréatique bovine avec une activité désoxyribonucléase et une thermolabilité accrue comparées à la référence de type sauvage, moyennant quoi l'acide aminé différent est choisi dans le groupe constitué par

- Ala, Ser, Thr, Gly, et Val lorsque l'acide aminé différent remplace un résidu Cys173,Cys101 ou Cys104,
- Asp, Glu, Asn, G In, et Ile lorsque l'acide aminé différent remplace le résidu Lys117,
- His, Ala, Asn, et Gln lorsque l'acide aminé différent remplace un résidu Arg 185 ou Ara 187,
- Ala, Ser, T hr, Gly, et Val lorsque l'acide a miné différent remplace le résidu Ile3,
- Asn, Gln, et Ile lorsque l'acide aminé différent remplace un résidu Phe82 ou Phe128.

**2.** Variant de la désoxyribonucléase I pancréatique bovine selon la revendication 1, **caractérisé en ce que** l'activité désoxyribonucléase spécifique du variant de la désoxyribonucléase I pancréatique bovine est approximativement de zéro unité par mg de protéine après un chauffage du variant de la désoxyribonucléase I pancréatique bovine pendant environ 5 minutes à une température inférieure à 95 °C.

**3.** Procédé destiné à produire un variant de désoxyribonucléase I pancréatique bovine selon l'une quelconque des revendications 1 et 2, comprenant les étapes consistant à

(a) fournir un vecteur comprenant une séquence nucléotidique qui code pour le variant de la désoxyribonucléase I pancréatique bovine,
(b) transformer une souche hôte microbienne avec le vecteur,
(c) mettre en culture la souche hôte microbienne transformée dans un milieu de croissance qui contient des nutriments, moyennant quoi la souche hôte microbienne exprime le variant de la désoxyribonucléase I pancréatique bovine, et
(d) purifier le variant de la désoxyribonucléase I pancréatique bovine à partir de la souche hôte microbienne et/ou du milieu de croissance.

**4.** Procédé selon la revendication 3, **caractérisé en ce que** la séquence nucléotidique qui code pour le variant de la désoxyribonucléase 1 pancréatique bovine est SEQ ID NO: 3.

**5.** Procédé selon l'une quelconque des revendications 3 et 4, **caractérisé en ce que**

(a) le vecteur comprend une séquence nucléotidique qui code pour une préprotéine constituée du variant de la désoxyribonucléase I pancréatique bovine et d'un peptide signal,
(b) la souche hôte microbienne est une souche de levure méthylotrophique,
(c) le milieu de croissance contient du méthanol comme source de carbone,
(d) la souche de levure méthylotrophique exprime et sécrète le variant de la désoxyribonucléase 1 pancréatique bovine, et
(e) le variant de la désoxyribonucléase I pancréatique bovine est purifié à partir du milieu de croissance.

**6.** Procédé selon la revendication 5, **caractérisé en ce que** le peptide signal contient un site de clivage de signal peptidase qui est localisé au voisinage direct du premier acide aminé du variant de la désoxyribonucléase I pancréatique bovine.

**7.** Procédé selon l'une quelconque des revendications 5 et 6, **caractérisé en ce que** la séquence d'acides aminés de la préprotéine exprimée est choisie dans le groupe constitué par

(a) SEQ ID NO: 8,
(b) SEQ ID NO: 9, et
(c) SEQ ID NO: 10.

**8.** Procédé selon l'une quelconque des revendications 5 à 7, **caractérisé en ce que** la séquence nucléotidique codant pour le variant de la désoxyribonucléase I pancréatique bovine est SEQ ID NO: 3.

**9.** Procédé selon l'une quelconque des revendications 5 à 8, **caractérisé en ce que** la séquence nucléotidique codant pour la préprotéine est constituée de la séquence nucléotidique codant pour le peptide signal fusionné à la séquence nucléotidique codant pour le variant de la désoxyribonucléase I pancréatique bovine.

**10.** Procédé selon l'une quelconque des revendications 5 à 9, **caractérisé en ce que** la séquence nucléotidique codant pour le peptide signal est choisie dans le groupe constitué par

(a) SEQ ID NO: 5,
(b) SEQ ID NO: 6, et
(c) SEQ ID NO: 7.

**11.** Procédé selon l'une quelconque des revendications 5 à 10, **caractérisé en ce que** la séquence nucléotidique codant pour la préprotéine est liée de manière opérationnelle à un promoteur ou un élément promoteur.

**12.** Procédé selon l'une quelconque des revendications 5 à 11, **caractérisé en ce que** la souche de levure méthylotrophique est une espèce Hansenula, Pichia, Candida ou Torulopsis.

**13.** Procédé selon la revendication 12, **caractérisé en ce que** la souche de levure méthylotrophique est choisie dans le groupe constitué par *Pichia pastoris, Hansenula polymorpha, Candida boidinii* et *Torulopsis glabrata.*

**14.** Procédé selon la revendication 13, **caractérisé en ce que** la souche de levure méthylotrophique est la souche *Pichia pastoris* avec le numéro d'accession de l'American Type Culture Collection 76273 ou bien un dérivé de celle-ci.

**15.** Souche de *Pichia pastoris* avec un chromosome qui contient un vecteur comprenant une séquence nucléotidique qui code pour une préprotéine constituée du variant d e la désoxyribonucléase I pancréatique bovine et d'un peptide signal, liée de manière opérationnelle au promoteur AOX1 de *Pichia pastoris* selon SEQ ID NO: 11 ou un élément promoteur de celle-ci,
moyennant quoi la séquence nucléotidique qui code pour la préprotéine est SEQ ID NO: 6 ou SEQ ID NO: 7, fusionnée à SEQ ID NO: 3.

**16.** Utilisation d'un variant de la désoxyribonucléase I pancréatique bovine selon l'une quelconque des revendications 1 et 2
pour hydrolyser l'ADN et réduire par la suite l'activité désoxyribonucléase spécifique du variant de la désoxyribonucléase I pancréatique bovine à approximativement zéro unité par mg de protéine par un chauffage du variant de la désoxyribonucléase I pancréatique bovine pendant environ 5 minutes à une température inférieure à 95 °C.

**17.** Utilisation d'un variant de la désoxyribonucléase I pancréatique bovine selon la revendication 16, **caractérisée en ce que**
l'activité désoxyribonucléase spécifique du variant de la désoxyribonucléase I pancréatique bovine est réduite à approximativement zéro unité par mg de protéine par un chauffage du variant de la désoxyribonucléase pancréatique bovine pendant environ 5 minutes à une température d'approximativement 70 °C.

**18.** Kit de parties contenant le variant de la désoxyribonucléase I pancréatique bovine selon l'une quelconque des revendications 1 et 2 et un tampon de réaction comprenant un cation divalent.

**19.** Kit de parties selon la revendication 18, **caractérisé en ce que**
le variant de la désoxyribonucléase I pancréatique bovine est dissous dans un tampon contenant 2 mM de TrisHCl, 2 mM de $MgCl_2$, 4 mM de $CaCl_2$, 50 % de glycérol, pH 7,6, et le tampon de réaction concentré dix fois contient 100 mM de TrisHCl, pH 7,5, 100 mM de $MgCl_2$, et 10 mM de dithioérythritol.

Figure 1:

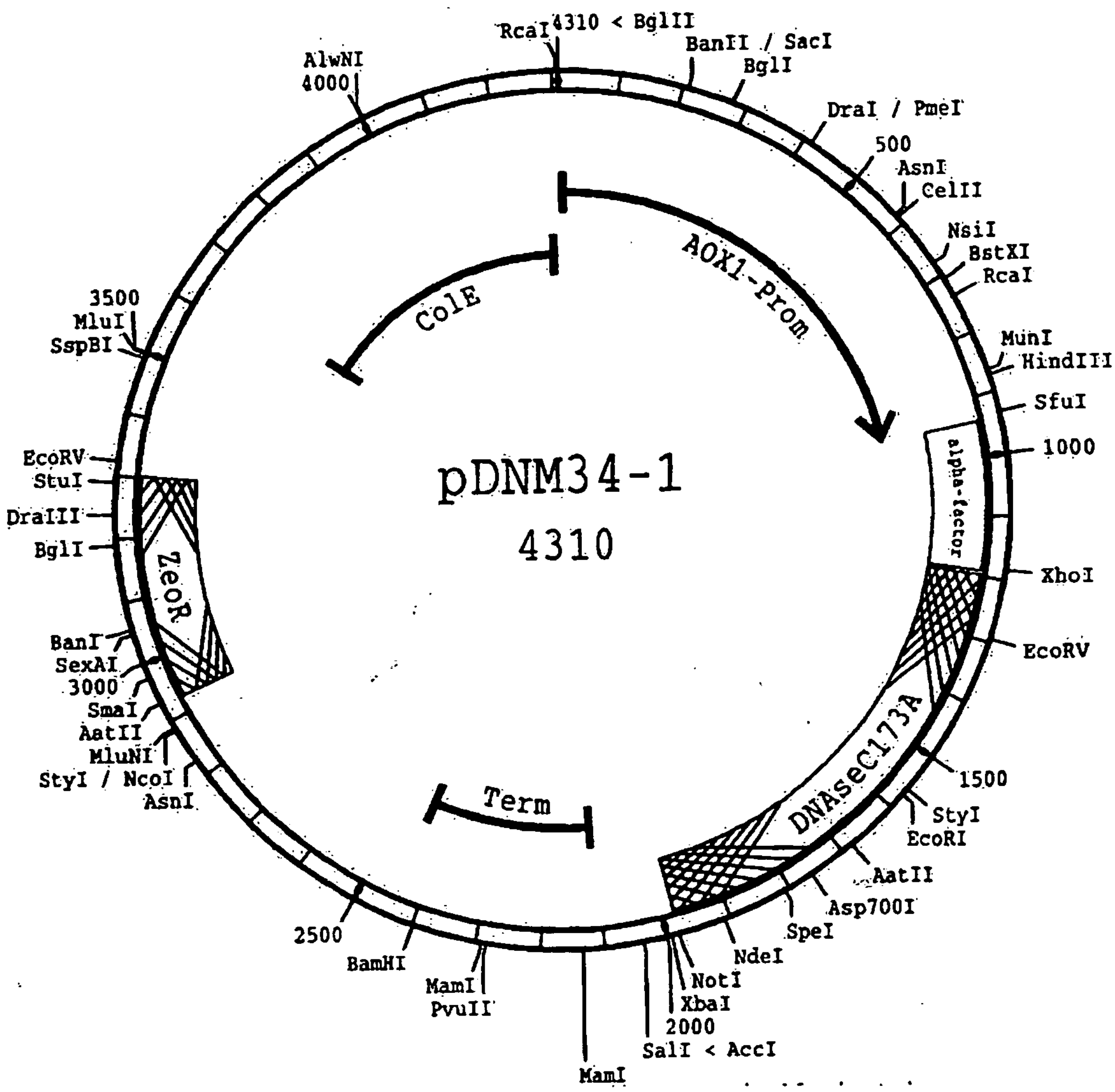
pDNM34-1
4310
RcaI
4310 < BglII
BanII / SacI
BglI
DraI / PmeI
500
AsnI
CelII
NsiI
BstXI
RcaI
MunI
HindIII
SfuI
1000
alpha-factor
XhoI
EcoRV
DNAseC173A
1500
StyI
EcoRI
AatII
Asp700I
SpeI
NdeI
NotI
XbaI
2000
SalI < AccI
MamI
MamI
PvuII
BamHI
2500
AsnI
StyI / NcoI
MluNI
AatII
SmaI
3000
SexAI
BanI
BglI
DraIII
StuI
EcoRV
ZeoR
SspBI
MluI
3500
4000
AlwNI
ColE
AOX1-Prom
Term

Figure 2:

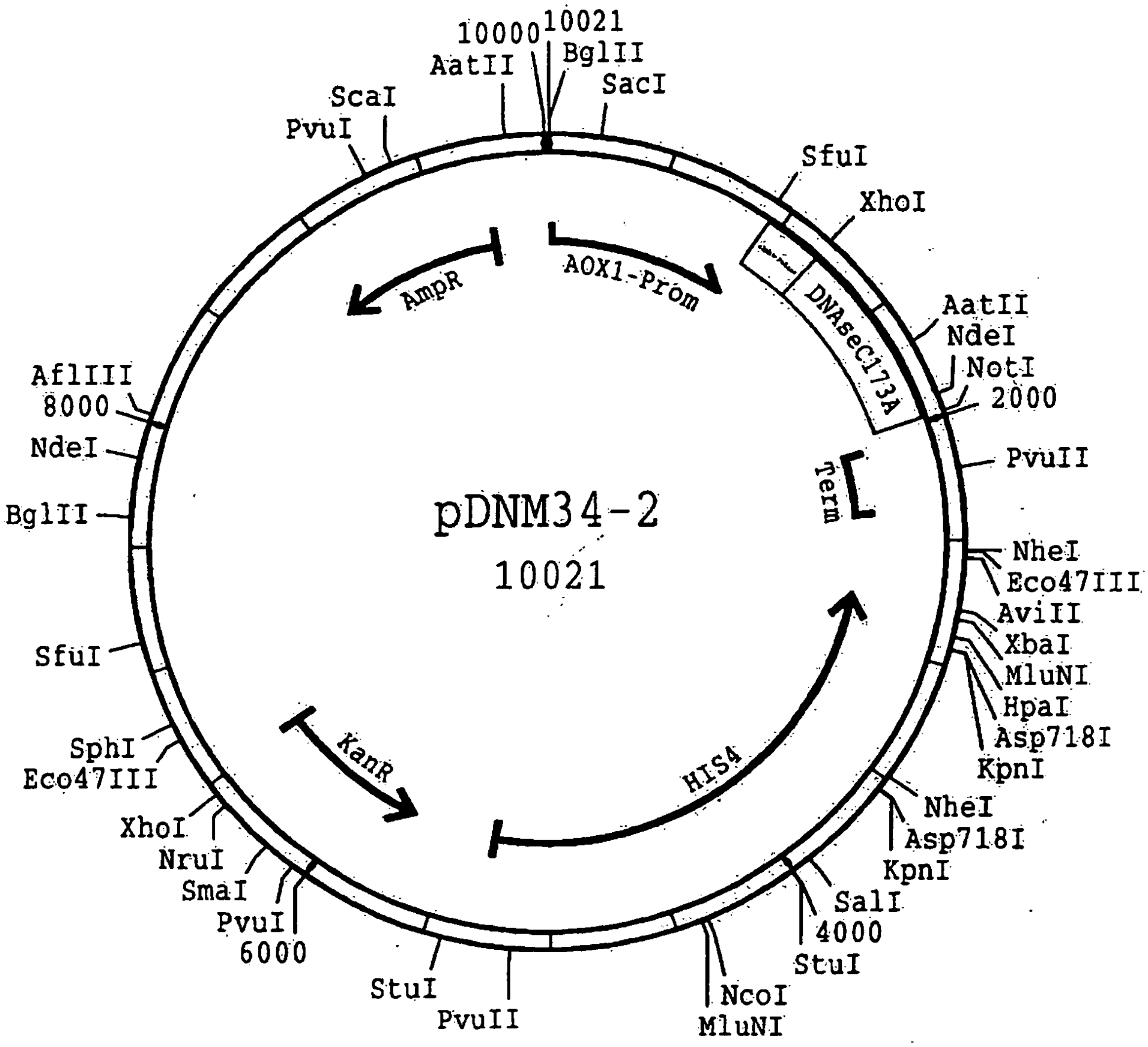
pDNM34-2
10021
10000
10021
AatII
BglII
SacI
ScaI
PvuI
SfuI
XhoI
AOX1-Prom
AmpR
DNAseC173A
AatII
NdeI
NotI
2000
PvuII
Term
NheI
Eco47III
AviII
XbaI
MluNI
HpaI
Asp718I
KpnI
NheI
Asp718I
KpnI
SalI
4000
StuI
NcoI
MluNI
HIS4
KanR
PvuII
StuI
6000
PvuI
SmaI
NruI
XhoI
Eco47III
SphI
SfuI
BglII
NdeI
8000
AflIII

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20020042052 A1
- US 5618676 A
- US 5854018 A
- US 5856123 A
- US 5919651 A
- EP 0116201 A
- US 4870008 A
- US 4683293 A
- US 4808537 A
- US 4812405 A
- US 4818700 A
- US 4837148 A
- US 4855231 A
- US 4857467 A
- US 4879231 A
- US 4882279 A
- US 4885242 A
- US 4895800 A
- US 4929555 A
- US 5002876 A
- US 5004688 A
- US 5032516 A
- US 5122465 A
- US 5135868 A
- US 5166329 A
- WO 0056903 A
- US 5324639 A


### Non-patent literature cited in the description


- **SAMBROOK ; FRITSCH ; MANIATIS.** Molecular Cloning, A Laboratory Manual. CSHL Press, 2001
- **KUNITZ, M.** *J. Gen. Physiol.,* 1950, vol. 33, 349-362 363
- Molecular Biology of the Cell. Garland Science Publishing, 2002
- **WATERS et al.** *J. Biol. Chem.,* 1988, vol. 263, 6209-14
- **HANAKI K. et al.** *Biotechniques,* 2000, vol. 29, 38-42
- **BICKLER et al.** *Biotechniques,* 1992, vol. 13, 64-66
- **WANG W.-Y. et al.** *Biochem J.,* 2000, vol. 346, 799-804
- **SUCK, D. et al.** *Nature,* 1988, vol. 332, 464-468
- **LAHM, A. ; SUCK, D.** *J. Mol. Biol.,* 1991, vol. 221, 645-667
- **JULIUS, D. et al.** *Cell,* 1984, vol. 37, 1075-1089
- **WERTEN, M.W. et al.** *Yeast,* 1999, vol. 15, 1087-1096
- **THILL, G.P. et al.** Positive and Negative Effects of Multi-Copy Integrated Expression in Pichia pastoris. *International Symposium on the Genentics of Microorganisms,* 1990, vol. 2, 477-490
- **VEDVICK, T. et al.** *J. Ind. Microbiol.,* 1991, vol. 7, 197-201
- **DROCOURT, D. et al.** *Nucleic Acids Res.,* 1990, vol. 18, 4009
- **CARMELS, T. et al.** *Curr. Genet.,* 1991, vol. 20, 309-314
- **SOUTHERN, P.J. ; BERG, P.** *J. Mol. Appl. Genet.,* 1982, vol. 1, 327-341
- **VAN TREECK, U. et al.** *Antimicrob Agents Chemother.,* 1981, vol. 19, 371-380
- **BECK, E. et al.** *Gene,* 1982, vol. 19, 327-336
- **CHEN, X.J. ; FUKUHARA, H.** *Gene,* 1988, 181-192
- **FUNAKOSHI, A. et al.** *J. Biochem.,* 1980, vol. 88, 1113-1138
- **PAUDEL, H.K. ; LIAO, T.H.** *J. Biol Chem.,* 1986, vol. 261, 16006-16011
- **NEFSKY, B. ; BRETSCHER, A.** *Eur. J. Biochem.,* 1989, vol. 179, 215-219